# EUROPEAN PATENT APPLICATION

(11) **EP 2 682 389 A1**
(43) Date of publication of application: **08.01.2014**
(21) Application number: 12305793.7
(22) Date of filing: 02.07.2012
(51) Int. Cl.: C07D 239/22, C07D 405/04, C07D 405/12, C07D 405/14, C07D 409/04, A61K 31/505, A61P 35/00

(54) **Dihydropyrimidin-2(1H)-ones and dihydropyrimidin-2(1H)-thiones as inhibitors of sodium iodide symporter**

(71) Applicant: COMMISSARIAT A L'ENERGIE ATOMIQUE ET AUX ENERGIES ALTERNATIVES, 75015 Paris (FR)
(72) Inventor: Ambroise, Yves, 91940 Les Ulis (FR); Lacotte, Pierre, 75014 Paris (FR)
(74) Representative: Mena, Sandra

(57) **Abstract**

The invention relates to novel dihydropyrimidin-2(1H)-ones and dihydropyrimidin-2(1H)-thiones of formula (I): 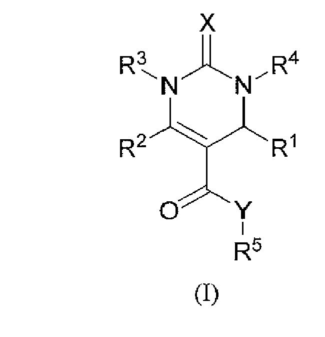 for use as medicaments, and in particular as inhibitors of sodium iodide symporter (NIS) and reducers of iodine transport and/or accumulation into NIS-expressing cells. The invention also relates to a pharmaceutical composition comprising at least one compound of formula (I) as active principle. Finally, the present invention relates to specific dihydropyrimidin-2(1H)-ones and dihydropyrimidin-2(1H)-thiones of formula (I) as such.

## Description

The invention relates to novel dihydropyrimidin-2(1H)-ones and dihydropyrimidin-2(1H)-thiones of formula (I) for use as medicaments, and in particular as inhibitors of sodium iodide symporter (NIS) and reducers of iodine transport and/or accumulation into NIS-expressing cells. The invention also relates to a pharmaceutical composition comprising at least one compound of formula (I) as active principle. Finally, the present invention relates to specific dihydropyrimidin-2(1H)-ones and dihydropyrimidin-2(1H)-thiones of formula (I) as such.

The translocation of iodide from blood into the thyroid gland is an essential step for the biosynthesis of thyroid hormones T3 and T4 which are responsible of many vital mechanisms in vertebrates such as metabolism regulation and central nervous system development (S. P. Porterfield et al., Endocr. Rev., 1993, 14, 94-106). This transport is mediated by the sodium iodide symporter (NIS), an integral membrane glycoprotein located at the basolateral side of thyrocytes. NIS is an integral plasma membrane glycoprotein that mediates active I⁻ transport into the thyroid follicular cells, the first step in thyroid hormone biosynthesis. NIS-mediated thyroidal I- transport from the blood-stream to the colloid is a vectorial process made possible by the selective targeting of NIS to the basolateral membrane. The molecular characterization of NIS was carried out after cloning the rat and human forms in 1996 (G. Dai, Nature, 1996, 379, 458-460; P. A. Smanik, Biochem. Biophys. Res. Commun., 1996, 226, 339-345). NIS is essentially expressed in thyroid follicular cells and also in several other tissues including the salivary glands, gastric mucosa, and the lactating mammary glands. NIS provides the basis for the effective diagnostic and therapeutic management of thyroid cancer and its metastases with radioiodide. Clinically related topics include the analysis of congenital I- transport defect-causing NIS mutations and the role of NIS in thyroid cancer. NIS has been transduced into various kinds of cancer cells to render them susceptible to destruction with radionucleide.

Other monovalent anions such as ClO₄⁻, SCN⁻, BF₄⁻, PF₆⁻, NO₃⁻ can also be transported by NIS (J. Wolff, Physiol. Rev., 1964, 44, 45-90; P. A. Jones, Toxicology in vitro, 1996, 10, 149-160). They provoke a competitive inhibition of iodide transport in rat thyroid-derived cells (FRTL5) with IC₅₀ values of 0.14, 14, 0.75, 0.009, and 250 µM, respectively (F. Waltz et al., Anal. Biochem., 2010, 396, 91-95). Thorough biochemical analysis has clarified the mechanism of iodide uptake and revealed the key role of NIS in many thyroid as well as extra-thyroid diseases such as cancer (thyroid, breast...) (T. Kogai et al., Endocr. Relat. Cancer, 2006, 13, 797-826), autoimmune diseases (Hashimoto and Basedow-Graves' diseases), toxic nodules, thyroiditis, multinodular goiter, etc. (O. Dohan et al., Endocr. Rev., 2003, 24, 48-77). The prevalence rate of these thyroid-related disorders is close to 7% in Western countries. In case of nuclear accident, the entrapment of radioactive isotopes of iodide by the thyroid gland is a major source of concern since this accumulation is directly responsible for an increase of cancer incidence. A dramatic example of this is the Tchernobyl accident in 1986 after which the World Health Organization (WHO) predicted that 9,000 individuals would die from cancer as a direct result of this disaster. Recent events in Fukushima have reminded us how tragic are the consequences of a nuclear reactor breakdown and thus, how important and urgent it is to find solutions to prevent and treat radioactive contamination. One solution is to develop radioprotective small molecules capable of blocking radioiodide uptake and/or best, enabling chemoremediation after the contamination has occurred. On the other hand, the ability of the thyroid gland to accumulate radioiodine has long provided the basis for the diagnosis and treatment of thyroid disorders (E. L. Mazzaferri, The thyroid: a fundamental and clinical text 7th ed.; Braverman, L. E.; Utiger R. D. Eds; Lippincott-Raven: Philadelphia, 1996; pp. 922-945). It is today proposed to extend this strategy to extra-thyroid tissue for the diagnosis and destruction of cancer cells by ¹³I after targeted NIS gene transfer (D. P. Carvalho et al., Arq. Bras. Endocrinol. Metabol., 2007, 51, 672-682; C. Spitzweg et al., Clin. Endocrinol., 2002, 57, 559-574). In this case, compounds increasing radioiodide retention in NIS-expressing cell would be very useful to ensure strong and specific toxic effect (N. Lecat-Guillet et al., ChemMedChem, 2008, 3, 1211-1216; T. Kogai et al., Endocr. Relat., Cancer, 2006, 13, 797-826). Small molecules affecting NIS function are unique tools for the study and treatment of many thyroid as well as non-thyroid dysfunctions.

Recently, a high throughput screening led to the discovery of new potent iodide transport blockers **(ITB1** to **ITB10,** Scheme 1) (N. Lecat-Guillet et al., ChemBioChem, 2008, 9, 889-895). These compounds showed rapid and total inhibition of iodide transport using isotopic flux measurement in human embryonic kidney cells stably expressing the human NIS (hNIS-HEK293) as well as in rat thyroid-derived cell lines (FRTL5) with inhibitory concentration values (IC₅₀) in the nano- and micromolar ranges. This inhibition was further confirmed by measurement of iodide-induced current in hNIS-expressing oocytes from *Xenopus laevis* (Lindenthal et al., J. Endocrinol., 2009, 200, 357-365). Among the 10 ITBs **(ITB1-10),** 3,4-dihydropyrimidin-2(1*H*)-one, Compound **1** (**ITB9,** see Scheme 1), was shown to be the most promising NIS inhibitor. The IC₅₀ value of Compound 1 was reported to be 0.4 µM in FRTL5 cells. Further analysis showed that Compound 1 can trigger a rapid efflux of iodide from preloaded hNIS-HEK293 cells, and was not found cytotoxic at concentration up to 200 µM. The discovery of Compound 1 as a powerful iodide uptake inhibitor is particularly attractive because dihydropyrimidin-2(1*H*)-ones are small versatile structures which can be easily synthesized at low cost and on a large scale. An additional small molecule **(ITB11,** Scheme 1) was later identified as an iodide uptake inhibitor in FRTL5 cells with an IC₅₀ value of 0.4 µM (N. Lecat-Guillet et al., ChemMedChem, 2008, 3, 1207-1209). **ITB11** was discovered from rational design, because it shares structural similarities with the NIS substrate BF₄⁻.

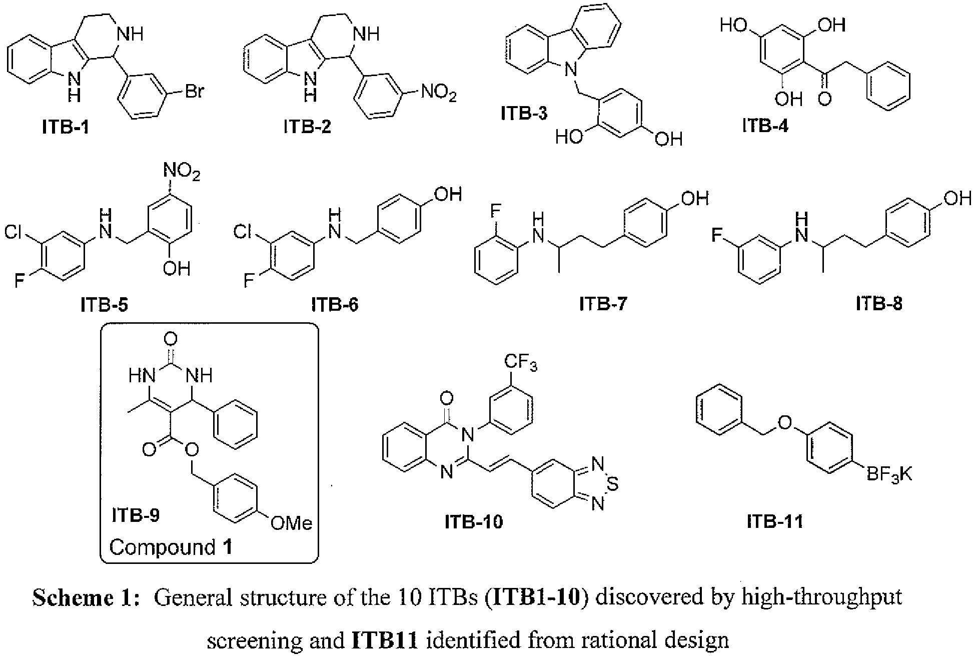

The inventors have now surprisingly discovered a new class of analogs of 3,4-dihydropyrimidin-2(1*H*)-ones with improved effects on iodide uptake in FRTL5 cells by measuring their IC₅₀ values. This new class of compounds present a strong enhancement of activity compared to Compound **1.** The compounds of the invention are thus more suitable for *in vivo* application.

Besides, no chemical compounds are currently available to combat contamination with radioisotopes of iodine, against the adverse effects of an over-accumulation of cold iodine (in some cases of hyperthyroidism and thyrotoxicosis), and for use in functional imaging of NIS.

A first subject of the invention is therefore a compound of general formula (I) below: 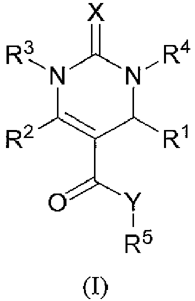 or a pharmaceutically acceptable salt thereof, wherein:
● X = O or S,
● Y = O or NH, and preferably Y = O,
● R₁, R₂, R₃, R₄ and R₅, identical or different, are selected from hydrogen, C₁-C₂₀ linear or branched alkyl, alkoxy, alkenyl, alkynyl, cycloalkyl, aryl, arylalkyl, heteroalkyl, heteroaryl and heteroarylalkyl groups, optionally substituted with one or more groups independently selected for example from halogen, hydroxyl, cyano, nitro, carboxylate, carboxyester, amino, C₁-C₁₂ alkylamino, C₁-C₁₂ aryl and C₁-C₁₂ alkoxy groups,
with the proviso that when X = O, Y = O, R₁ is a phenyl group, R₂ is a methyl group, R₃ and R₄ are hydrogen, R₅ is other than a 4-methoxybenzyl group,
for use as a medicament.

In the sense of the present invention:
- Alkyl groups are chosen among (C₁-C₂₀)alkyl groups, and preferably (C₁-C₆)alkyl groups such as methyl, ethyl, n-propyl, isopropyl, n-butyl, *sec-*butyl, *tert*-butyl and isobutyl radicals;
- Cycloalkyl groups refer to a monovalent cyclic hydrocarbon radical preferably of 3 to 7 ring carbons. The cycloalkyl group can have one or more double bonds and can optionally substituted. The term "cycloalkyl" includes, for examples, cyclopropyl, cyclohexyl, cyclohexenyl and the like;
- Heteroalkyl groups mean alkyl groups as defined above in which one or more hydrogen atoms to any carbon of the alkyl is replaced by a heteroatom selected from the group consisting ofN, O, P, B, S, Si, Sb, Al, Sn, As, Se and Ge. The bond between the carbon atom and the heteroatom may be saturated or unsaturated. Suitable heteroalkyl groups include cyano, benzoyl, methoxy, acetamide, borates, sulfones, sulfates, thianes, phosphates, phosphonates, and the like;
- Alkoxy groups are chosen among (C₁-C₂₀)alkoxy groups, and preferably (C₁-C₄)alkoxy groups such as methyloxy, ethyloxy, n-propyloxy, iso-propyloxy, n-butyloxy, *sec-*butyloxy, *tert*-butyloxy and isobutyloxy radicals;
- Aryl groups means any functional group or substituent derived from at least one simple aromatic ring; an aromatic ring corresponding to any planar cyclic compound having a delocalized π system in which each atom of the ring comprises a p-orbital, said p-orbitals overlapping themselves. More specifically, the term aryl includes, but is not limited to, phenyl, biphenyl, 1-naphthyl, 2-naphtyl, anthracyl, pyrenyl, and the substituted forms thereof;
- Heteroaryl groups means any functional group or substituent derived from at least one aromatic ring as defined above and containing at least one heteroatom selected from P, S, O and N. The term heteroaryl includes, but is not limited to, furan, pyridine, pyrrole, thiophene, imidazole, pyrazole, oxazole, isoxazole, thiazole, isothiazole, tetrazole, pyridazole, pyridine, pyrazine, pyrimidine, pyridazine, benzofurane, isobenzofurane, indole, isoindole, benzothiophene, benzo[c]thiophene, benzimidazole, indazole, benzoxazole, benzisoxazole, benzothiazole, quinoline, isoquinoline, quinoxaline, quinazoline, cinnoline, purine and acridine. The aryl and heteroaryl groups of the invention comprise preferably 1 to 12 carbon atoms, and more preferably 5 or 6 carbon atoms;
- Arylalkyl means any group derived from an alkyl group as defined above wherein a hydrogen atom is replaced by an aryl or an heteroaryl group.

According to the invention, halogen atoms are chosen among bromine, chlorine, fluorine and iodine, and preferably bromine, chlorine and fluorine.

According to a preferred embodiment, R₁ is selected from C₁-C₆ linear or branched alkyl, cycloalkyl, arylalkyl, heteroalkyl, heteroaryl and heteroarylalkyl groups, optionally substituted with one or more groups independently selected from halogen, hydroxyl, cyano, nitro and C₁-C₇ alkoxy groups, and preferably R₁ is selected from optionally substituted C₁-C₆ cycloalkyl, furane, thiophene, pyrrole, pyrazole, oxadiazole, oxazole, isoxazole, thiazole, isothiazole, and phenyl substituted with at least one halogen, and preferably R₁ is a furane.

According to another preferred embodiment, R₂ is selected from C₁-C₆ linear or branched alkyl, phenyl, -CH₂-O-phenyl, benzyl, thiophene and -CH₂-thiophene.

According to another preferred embodiment, R₃ is selected from hydrogen and C₁-C₆ linear or branched alkyl, and preferably R₃ is hydrogen or a methyl group.

According to another preferred embodiment, R₄ is selected from hydrogen and C₁-C₆ linear or branched alkyl, and preferably R₄ is hydrogen or a methyl group.

According to another preferred embodiment, R₅ is a benzyl group optionally substituted with one or more groups independently selected from halogen, methyl, hydroxyl, cyano, nitro and C₁-C₇ alkoxy groups, and preferably R₅ is a methoxybenzyl group or a benzodioxolylmethyl group, such as piperonyl group.

As particular compounds of formula (I) above, we can mentioned the following compounds:
- 4-methoxybenzyl 4-(furan-2-yl)-6-methyl-2-oxo-1,2,3,4-tetrahydropyrimidine-5-carboxylate: 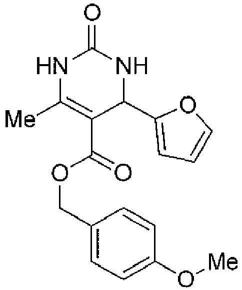
- 4-methoxybenzyl 4-(furan-2-yl)-1,3,6-trimethyl-2-oxo-1,2,3,4-tetrahydropyrimidine-5-carboxylate: 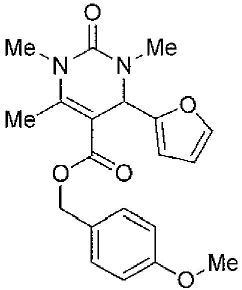
- 3-methoxybenzyl 4-(furan-2-yl)-6-methyl-2-oxo-1,2,3,4-tetrahydropyrimidine-5-carboxylate: 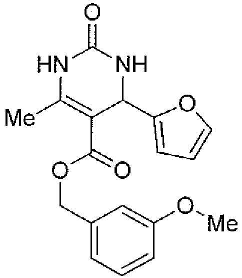
- 3-methoxybenzyl 4-(furan-2-yl)-1,3,6-trimethyl-2-oxo-1,2,3,4-tetrahydropyrimidine-5-carboxylate: 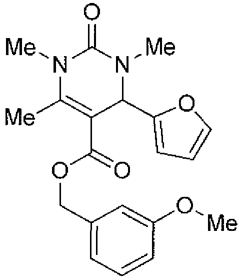
- benzo[d][1,3]dioxol-5-ylmethyl 4-(furan-2-yl)-6-methyl-2-oxo-1,2,3,4-tetrahydropyrimidine-5-carboxylate: 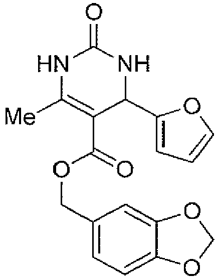
- benzo[d][1,3]dioxol-5-ylmethyl 4-(furan-2-yl)-1,3,6-trimethyl-2-oxo-1,2,3,4-tetrahydropyrimidine-5-carboxylate:

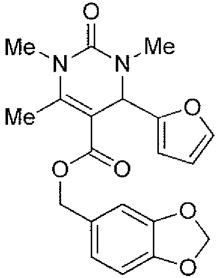

More particularly, the invention concerns the compound of formula (I) for use as a medicament:
- for the inhibition of sodium iodide symporter (NIS), and the reduction of iodine transport and/or accumulation into NIS-expressing cells,
- for the *in vivo* diagnosis of NIS pathologies by functional imaging,
- for radioiodide decontamination of humans or animals after exposure to radioactive iodine species,
- for the prevention and/or the treatment of thyroid disorders, and more particularly of hyperthyroidism triggered by iodine overload, thyrotoxicosis, thyroiditis and toxic nodular goiter,
- for the prevention and/or the treatment of cancers, and more particularly of thyroid and breast cancers,
- for the prevention and/or the treatment of autoimmune diseases, and more particularly of Hashimoto and Basedow-Graves' diseases.

Functional imaging of NIS is a method of detecting or measuring changes in the spatial distribution of NIS within the body. To achieve this, the compound of formula (I) needs to be derivatized into a probe with similar chemical and biological characteristics, plus a chemical tag for detection. For the chemical tag, it is generally used radioisotopes such as carbon-11, nitrogen-13, oxygen-15 and fluorine-18, for use in Positron Emission Tomography (PET); technetium-99m for use in Single-Photon Emission Computed Tomography (SPECT).

Another subject matter of the invention is a pharmaceutical composition comprising at least one compound of formula (I) of the invention as an active principle, and at least one pharmaceutically acceptable excipient.

The expression "pharmaceutically acceptable excipient" refers to any diluents, adjuvants or vehicles, such as preserving agents, fillers, disintegrating agents, wetting agents, emulsifying agents, suspending agents, solvents, dispersion media, coatings, antibacterial and antifungal agents, isotonic and absorption delaying agents and the like.

The pharmaceutical composition of the present invention may be administered by any suitable route, for example, by oral, buccal, inhalation, sublingual, nasal, percutaneous, *i.e.* transdermal or parenteral (including intravenous, intramuscular, subcutaneous and intracoronary) administration. Therefore, the pharmaceutical composition of the invention can be provided in various forms, such as in the form of hard gelatin capsules, of capsules, of compressed tablets, of suspensions to be taken orally, of lozenges or of injectable solutions or in any other form appropriate to the method of administration.

The pharmaceutical composition according to the invention includes those wherein a compound of formula (I) is administered in an effective amount to achieve its intended purpose. Determination of the effective amounts is well within the capability of those skilled in the art.

A "therapeutically effective dose" refers to that amount of compound of formula (I) which results in achieving the desired effect. Toxicity and therapeutic efficacy of compound of formula (I) can be easily determined by standard pharmaceutical procedures in cell cultures or experimental animals, *i.e*. for determining the LD₅₀ (the dose lethal to 50% of the population) and the ED₅₀ (the dose therapeutically effective in 50% of the population). The dose ratio between toxic and therapeutic effects is the therapeutic index, which is expressed as the ratio between LD₅₀ and ED₅₀. The data obtained from such data can be used in formulating range of dosage for use in humans. The dosage of compound of formula (I) preferably lies within a range of circulating concentrations that include the ED₅₀ with little or no toxicity. The dosage can vary within this range depending upon the dosage form employed, and the route of administration.

The exact formulation, route of administration, and dosage can be chosen by the individual physician in view of the patient's conditions. Dosage amount and interval of administration can be adjusted individually to provide plasma levels of compound of formula (I) which are sufficient to maintain the preventive or therapeutic effects.

The amount of pharmaceutical composition administered will therefore depend on the subject being treated, on the subject's weight, the severity of the affliction and the manner of administration.

For human and other mammal use, the compounds of formula (I) can be administered alone, but they are preferably administered in admixture with at least one pharmaceutically acceptable carrier, the nature of which will depend on the intended route of administration and the presentation form. Pharmaceutical composition for use according to the present invention thus can be formulated in a conventional manner using one or more physiologically acceptable carriers comprising one or more excipient(s) and/or auxiliary(ies) that facilitate processing of the compounds of formula (I) into preparations which can be used pharmaceutically. Amongst the excipients and auxiliaries which can be used in the pharmaceutical composition according to the invention, one can mention anti-agglomerating agents, preservatives agents, dyes, vitamins, inorganic salts, taste-modifying agents, smoothing agents, coating agents, isolating agents, stabilizing agents, wetting agents, anti-caking agents, dispersing agents, emulsifying agents, aromas, penetrating agents, solubilizing agents, etc., mixtures thereof and generally any excipient conventionally used in the pharmaceutical industry.

By way of example, when the pharmaceutical composition is administered orally, the carrier may comprise one or several excipients such as talc, lactose, starch or modified starches, cellulose or cellulose derivatives, polyethylene glycols, acrylic acid polymers, gelatin, magnesium stearate, animal or vegetal fats of natural or synthetic origin, paraffin derivatives, glycols, etc.

For general information about the formulation and administration of pharmaceutical compositions, one can obviously refer to the book "Remington's Pharmaceutical Sciences", last edition. Of course, a person skilled in the art will take care on this occasion that the excipient(s) and/or auxiliary(ies) optionally used are compatible with the intrinsic properties attached to the pharmaceutical composition in accordance with the invention.

These pharmaceutical compositions can be manufactured in a conventional manner, *i.e.* by conventional mixing, dissolving, granulating, dragee-making, emulsifying, encapsulating, entrapping or lyophilizing processes. Proper formulation is dependent upon the route of administration chosen.

Finally, the invention also concerns compounds of general formula (I) as such, said compounds responding to the general formula (I) below: 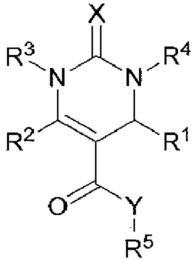 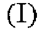 or a pharmaceutically acceptable salt thereof, wherein:
● X = O or S,
● Y = O or NH,
● R₁, R₂, R₃, R₄ and R₅, identical or different, are selected from hydrogen, C₁-C₂₀ linear or branched alkyl, alkoxy, alkenyl, alkynyl, cycloalkyl, aryl, arylalkyl, heteroalkyl, heteroaryl and heteroarylalkyl groups, optionally substituted with one or more groups independently selected for example from halogen, hydroxyl, cyano, nitro, carboxylate, carboxyester, amino, C₁-C₁₂ alkylamino, C₁-C₁₂ aryl and C₁-C₁₂ alkoxy groups,
with the proviso that:
- when X = O, Y = O, R₁ is a phenyl group, R₂ is a methyl group, R₃ and R₄ are hydrogen, R₅ is other than hydrogen, a benzyl group, a piperonyl group, an ethyl group, a furan-2-ylmethyl group or a 4-methoxybenzyl group, or
- when X = S, Y = O, R₁ is a phenyl group, R₂ is a methyl group, R₃ and R₄ are hydrogen, R₅ is other than hydrogen or a propen-2-yl group.

In addition to the above provisions, the invention also comprises other provisions which will become clear from the description which follows, which refers to examples evaluating the effects of structural variations of compounds of formula (I) on iodide uptake in FRTL5 cells by measuring the IC₅₀ values of such compound.

### EXAMPLES:

### I- Method of synthesis

Most target 3,4-dihydropyrimidin-2(1*H*)-one (DHPM) 5-carboxy esters **(21-51** and **53-62)** were prepared according to the general Scheme 2 using the three-component ring-forming Biginelli reaction (P. Bignelli, Gazz. Chim. Ital., 1893, 23, 360-416; C. O. Kappe, Acc. Chem. Res., 2000, 33, 879-888). This reaction involves the condensation of an aldehyde, a β-ketoester and an urea or thiourea with catalytic acid.

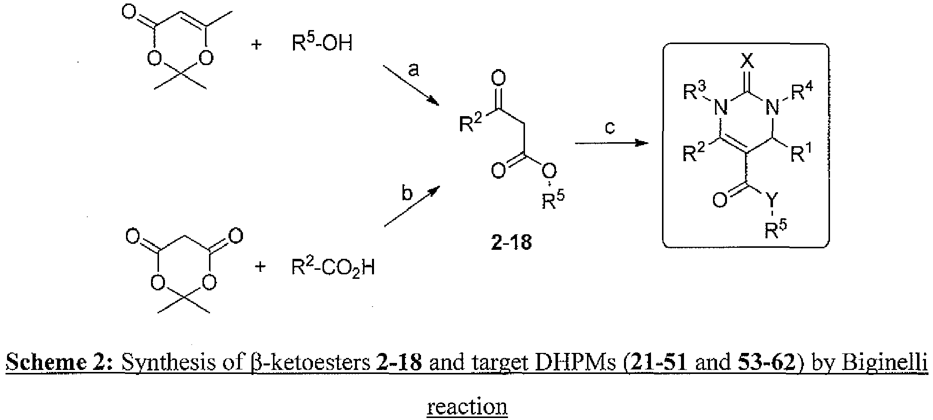

### Reagents and conditions: (a) AcOK, microwave, 120°C, 30 min, 26-97%; (b) DCC, DMAP, DCM, rt, 14h, then 4-methoxybenzyl alcohol, microwave, toluene, 100°C, 30 min, 50-75% (two steps); (c) method A: Yb(OTf)₃, solvent free, 100°C, 45 min - method B: Zn(OTf)₂, MeCN, reflux, 2-16h - method C: HCl, MeOH, 40°C, 1-3 days

The β-ketoesters used to prepare target compounds were obtained from commercial sources or synthesized by methods known in the literature. Acetoacetates **2-8** with variation at the ester R⁵ position were prepared from 2,2,6-trimethyl-4*H*-1,3-dioxin-4-one and diverse R⁵OH in the presence of potassium acetate (Scheme 2) (V. Sridharan et al., Synthesis, 2010, 6, 1053-1057). For β-ketoesters **9-18** with variations at the R² position, 2,2-dimethyl-1,3-dioxane-4,6-dione (Meldrum's Acid) was acetylated with diverse carboxylic acids (R²CO₂H) and the resulting intermediates were treated with 4-methoxybenzyl alcohol (Y. Oikawa et al., J. Org. Chem., 1978, 43, 2087-2088).

Three different Biginelli reaction conditions were used for the final ring-forming reaction: Yb(OTf)₃/solvent free (method A) (Y. Ma, J. Org. Chem., 2000, 65, 3864-3868), Zn(OTf)₂ in MeCN (method B) or HCl in H₂O/MeOH (method C). Biginelli reaction with monomethylurea provided selectively the N-1 methyl DHPMs **(50, 53, 56, 60).** This was verified by ¹H, ¹³C NMR and was in accord with other reports (O. Kappe, Tetrahedron, 1993, 49, 6937-6963). Some synthesized DHPMs also served as starting materials for the preparation of additional target compounds (Scheme 3). The carboxybenzyl ester **32** provided carboxylic acid **19** under hydrogenolytic conditions (H₂, Pd/C) (B. Desai et al., Tetahedron, 2006, 62, 4651-4664). The carboxamide derivative **63** was obtained by reacting Compound **19** with 4-piperonylamine in the presence of EDCI (1-ethyl-3-(3-dimethylaminopropyl) carbodiimide) or HBTU (O-Benzotriazole-N,N,N',N'-tetramethyl-uronium-hexafluorophosphate) as a coupling agent. The 3,4-dihydropyrimidin-2(1*H*)-thione **52** was prepared in a two step sequence by treatment of allyl ester **39** with Pd(PPh₃)₄ and diethylamine in tetrahydrofuran to provide the intermediate carboxylic acid **20** (B. Desai et al., Tetrahedron, 2006, 62, 4651-4664). This intermediate was subsequently reacted with 4-methoxybenzyl alcohol in EDCI/DMAP conditions to give DHPM **52.**

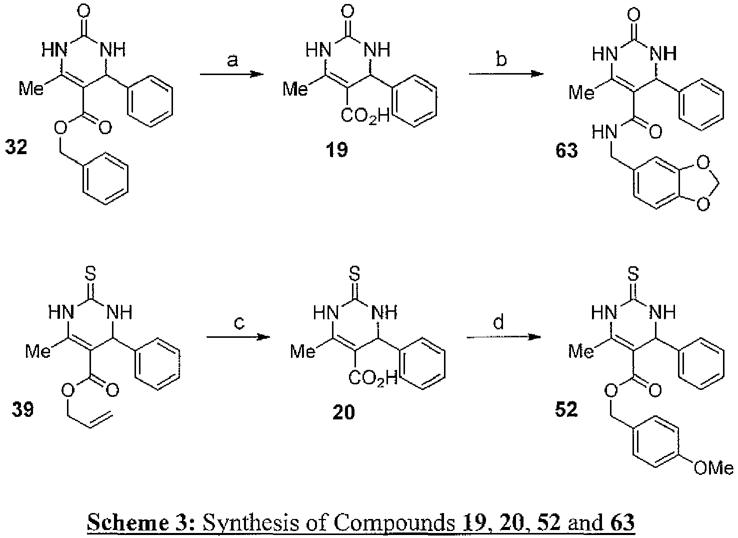

### Reagents and conditions: (a): H₂, Pd/C, MeOH, rt, 3.5 h, 79%; (b): 4-piperonylamine, HBTU, EDCI, DIEA, DMA, microwave, 80 °C, 30 min, 36%; (c): Pd(PPh₃)₄, DEA, THF, rt, 4 h, 35%; (d): 4-methoxybenzyl alcohol, EDCI, DMAP, DMA, 80 °C, 5 h, 18%.

The identity of the compounds was verified by MS, ¹H, ¹³C and NMR, and ¹⁹F NMR (when appropriate). The purity of all compounds tested was found to exceed 95% using a high-performance liquid chromatography (HPLC) system.

### II- Protocols for chemical synthesis

### II- 1) General methods for chemical syntheses and characterization

Reagents and solvents were from Sigma-Aldrich without further purification. Microwave-assisted reactions were run on a Discover SP system (CEM) equipped with an explorer module. Flash chromatography was performed on a CombiFlash Rf system (Teledyne Isco) using normal phase Redisep (Teledyne Isco) or SNAP (Biotage) cartridges. The HPLC-MS analysis was performed on a system equipped with a binary gradient solvent delivery system (LC-20AB, Shimadzu), a SIL-20A autosampler (Shimadzu) and a photodiode array detector (SPD-20A, Shimadzu). This system was coupled to an electrospray ionization Micromass-ZQ spectrometer (Waters) operating in both positive and negative mode. Each compound (8-15 µg) was applied to a 250 x 4.6 mm (5 µm) Zorbax SB-C18 (Agilent) equilibrated with acetonitrile/water = 30/70 (1 mL/min). Samples were eluted by increasing acetonitrile to 45% (10 min), then 85% (25 to 30 min). ¹H, ¹³C and ¹⁹F NMR spectra were recorded on a Bruker Avance DPX 400 spectrometer operating at 400 MHz (¹H), 100 MHz (¹³C) and 160 MHz (¹⁹F). The chemical shifts (δ) were expressed in ppm. Melting points (B-540, Büchi) are uncorrected.

### II- 2) General synthetic procedure and data analysis for intermediates 2-8

*General synthetic procedure:* 2,2,6-trimethyl-4*H*-1,3-dioxin-4-one (1.0 mL, 7.7 mmol) and alcohol (5.9 mmol) were mixed with potassium acetate (241 mg, 2.9 mmol) in a microwave vial. The mixture was microwaved for 20 minutes at 130 °C. Chromatography on silica gel (cHex/AcOEt) afforded **2-8.**

*4-Methoxybenzyl 3-oxobutanoate (**2**),* yellow liquid (73%). ¹H NMR (400 MHz, CDCl₃) *δ* 2.16 (s, 3H), 3.64 (s, 2H), 3.75 (s, 3H), 5.06 (s, 2H), 6.93 (d, *J* = 8.0 Hz, 2H), 7.31 (d, *J* = 8.0 Hz, 2H). ¹³C NMR (100 MHz, CDCl₃) *δ* 30.5, 50.0, 55.5, 66.3, 114.2, 128.1, 130.5, 159.6, 167.7, 202.2.

*2-Chlorobenzyl 3-oxobutanoate (**3**),* light-yellow liquid (79%). ¹H NMR (400 MHz, CDCl₃) *δ* 2.19 (s, 3H), 3.71 (s, 2H), 5.21 (s, 2H), 7.35-7.41 (m, 2H), 7.49-7.55 (m, 2H). ¹³C NMR (100 MHz, CDCl₃) *δ* 30.5, 49.9, 63.9, 127.8, 129.8, 130.6, 130.8, 133.1, 133.6, 167.4, 201.9.

*4-Chlorobenzyl 3-oxobutanoate (**4**),* light-yellow liquid (81%). ¹H NMR (400 MHz, CDCl₃) *δ* 2.18 (s, 3H), 3.69 (s, 2H), 5.13 (s, 2H), 7.41 (d, *J* = 8.4 Hz, 2H), 7.43 (d, *J* = 8.4 Hz, 2H). ¹³C NMR (100 MHz, CDCl₃) *δ* 30.6, 49.9, 65.6, 128.9, 130.3, 133.2, 135.3, 167.6, 202.0.

*4-Fluorobenzyl 3-oxobutanoate (**5**)*, yellow liquid (54%). ¹H NMR (400 MHz, CDCl₃) *δ* 2.17 (s, 3H), 3.67 (s, 2H), 5.12 (s, 2H), 7.19-7.24 (m, 2H), 7.43 (dd, *J* = 5.6, 8.4 Hz, 2H). ¹³C NMR (100 MHz, CDCl₃) *δ* 30.5, 49.9, 65.7, 115.7 (d, *J* = 21.3 Hz), 130.8 (d, *J* = 8.3 Hz), 132.5 (d, *J* = 3.0 Hz), 162.3 (d, *J* = 242.4 Hz), 167.6, 202.0. ¹⁹F NMR (160 MHz, CFCl₃):-114.0

*2-Methylbenzyl 3-oxobutanoate (**6**),* light-yellow liquid (86%). ¹H NMR (400 MHz, CDCl₃) *δ* 2.17 (s, 3H), 2.29 (s, 3H), 3.68 (s, 2H), 5.13 (s, 2H), 7.19-7.26 (m, 3H), 7.33 (d, *J* = 7.2 Hz, 1H). ¹³C NMR (100 MHz, CDCl₃) *δ* 18.8, 30.6, 49.9, 65.1, 126.3, 128.9, 129.6, 130.6, 134.1, 137.2, 167.6, 202.1.

*3-Methoxybenzyl 3-oxobutanoate (**7**),* yellow liquid (87%). ¹H NMR (400 MHz, CDCl₃) *δ* 2.24 (s, 3H), 3.49 (s, 2H), 3.80 (s, 3H), 5.14 (s, 2H), 6.84-6.92 (m, 3H), 6.90 (m, 1H). ¹³C NMR (100 MHz, CDCl₃) *δ* 30.4, 50.2, 55.5, 67.2, 113.9, 114.2, 120.6, 129.9, 137.0, 160.0, 167.1, 200.5.

*Benzo[d][1,3]dioxol-5-ylmethyl 3-oxobutanoate (**8**),* yellow liquid (91%). ¹H NMR (400 MHz, CDCl₃) *δ* 2.24 (s, 3H), 3.48 (s, 2H), 5.07 (s, 2H), 5.97 (s, 2H), 6.77-6.85 (m, 3H). ¹³C NMR (100 MHz, CDCl₃) *δ* 30.5, 50.0, 66.4, 101.5, 108.5, 109.3, 122.6, 129.9, 147.6, 147.7, 167.6, 202.0.

### II- 3) General synthetic procedure and data analysis for intermediates 9-18

*General synthetic procedure:* To a stirred solution of Meldrum's acid (1.0 mmol), carboxylic acid (1.0 mmol) and DCC (1.1 mmol) in dry CH₂Cl₂ (10 mL) was added DMAP (1.1 mmol) under argon atmosphere. After stirring one night at room temperature, DCU was filtered off and filtrate was concentrated under reduced pressure. The residue was dissolved in 20 mL ethyl acetate and washed with 10 mL 1M aq. HCl, 5 mL water and 5 mL brine. Organic layers were then dried on MgSO₄ and concentrated *in vacuo.* The residue was resuspended in 5 mL toluene and 4-methoxybenzyl alcohol (1.2 mmol) was added. The mixture was microwaved during 30 minutes at 100 °C. Evaporation of solvent and subsequent chromatography on silica gel (cHex/AcOEt) afforded title compounds **9-18.**

*4-Methoxybenzyl 3-oxopentanoate (**9**),* colorless liquid (72%). ¹H NMR (400 MHz, CDCl₃) *δ* 1.08 (t, *J* = 7.2 Hz, 3H), 2.55 (q, *J* = 7.2 Hz, 2H), 3.48 (s, 2H), 3.83 (s, 3H), 5.13 (s, 2H), 6.91 (d, *J* = 8.4 Hz, 2H), 7.32 (d, *J* = 8.8 Hz, 2H). ¹³C NMR (100 MHz, CDCl₃) *δ* 7.5, 36.3, 49.0, 55.3, 67.0, 114.0, 127.4, 130.2, 159.8, 167.2, 203.2.

*4-Methoxybenzyl 5-methyl-3-oxohexanoate (**10**),* yellow liquid (66%). ¹H NMR (400 MHz, CDCl₃) *δ* 0.91 (s, 3H), 0.93 (s, 3H), 2.12-2.17 (m, 1H), 2.39 (d, *J* = 6.8 Hz, 2H), 3.45 (s, 2H), 3.83 (s, 3H), 5.13 (s, 2H), 6.91 (d, *J* = 8.8 Hz, 2H), 7.32 (d, *J* = 8.8 Hz, 2H). ¹³C NMR (100 MHz, CDCl₃) *δ* 22.4, 24.3, 49.7, 51.8, 55.3, 66.9, 114.0, 127.4, 130.3, 159.8, 167.1, 202.3.

*4-Methoxybenzyl 3-oxooctanoate (11),* yellow liquid (70%). ¹H NMR (400 MHz, CDCl₃) *δ* 0.89 (t, *J* = 6.8 Hz, 3H), 1.24-1.30 (m, 4H), 1.56-1.60 (m, 2H), 2.50 (t, *J* = 7.4 Hz, 2H), 3.47 (s, 2H), 3.83 (s, 3H), 5.13 (s, 2H), 6.91 (d, *J* = 8.4 Hz, 2H), 7.32 (d, *J* = 8.4 Hz, 2H). ¹³C NMR (100 MHz, CDCl₃) *δ* 13.9, 22.4, 23.1, 31.1, 43.0, 49.3, 55.3, 66.9, 114.0, 127.4, 130.3, 159.8, 167.2, 202.8.

*4-Methoxybenzyl 3-cyclohexyl-3-oxopropanoate (**12**)*, yellow liquid (65%). ¹H NMR (400 MHz, CDCl₃) *δ* 1.20-1.30 (m, 5H), 1.66-1.69 (m, 1H), 1.77-1.84 (m, 4H), 2.37-2.44 (m, 1H), 3.52 (s, 2H), 3.83 (s, 3H), 5.13 (s, 2H), 6.91 (d, *J* = 8.8 Hz, 2H), 7.32 (d, *J* = 8.4 Hz, 2H). ¹³C NMR (100 MHz, CDCl₃) *δ* 25.5, 25.7, 28.2, 47.4, 50.8, 55.3, 66.9, 114.0, 127.5, 130.3, 159.7, 167.4, 205.8.

*4-Methoxybenzyl 3-oxo-3-phenylpropanoate (**13**),* mixture of tautomers, yellow liquid (51%). ¹H NMR (400 MHz, CDCl₃) *δ* 3.82 (s, 3H), 3.84 (s, 0.84H), 4.04 (s, 2H), 5.16 (s, 2H), 5.21 (s, 0.52H), 5.73 (s, 0.24H), 6.88 (d, *J*= 8.4 Hz, 2H), 6.94 (d, *J* = 8.8 Hz, 0.55H), 7.28 (d, *J*= 8.4 Hz, 2H), 7.37-7.45 (m, 1.32H), 7.47-7.50 (m, 2H), 7.59-7.63 (m, 1H), 7.78-7.80 (m, 0.52H), 7.93 (d, *J*= 8.4 Hz, 2H), 12.6 (s, 0.24H). ¹³C NMR (100 MHz, CDCl₃) *δ* 46.0, 55.3, 67.1, 113.9, 128.5, 128.6, 128.8, 130.2, 133.7, 135.9, 159.7, 167.4, 192.4.

*4-Methoxybenzyl 3-oxo-4-phenylbutanoate (**14**),* yellow liquid (66%). ¹H NMR (400 MHz, CDCl₃) *δ* 3.49 (s, 2H), 3.82 (s, 2H), 3.83 (s, 3H), 5.11 (s, 2H), 6.91 (d, *J*= 8.8 Hz, 2H), 7.18 (d, *J*= 6.8 Hz, 2H), 7.30-7.34 (m, 5H). ¹³C NMR (100 MHz, CDCl₃) *δ* 48.3, 50.0, 55.3, 67.0, 114.0, 127.3, 127.4, 128.6, 128.9, 130.3, 133.1, 159.8, 167.0, 200.3.

*4-Methoxybenzyl 3-oxo-4-phenoxybutaoate (**15**)*, colorless liquid (75%). ¹H NMR (400 MHz, CDCl₃) *δ* 3.69 (s, 2H), 3.82 (s, 3H), 4.62 (s, 2H), 5.14 (s, 2H), 6.84-6.88 (m, 4H), 7.03 (t, *J* = 7.4 Hz, 1H), 7.27-7.33 (m, 4H). ¹³C NMR (100 MHz, CDCl₃) *δ* 46.2, 55.3, 67.2, 72.4, 114.0, 114.5, 122.0, 127.2, 129.7, 130.3, 157.3, 159.8, 166.7, 200.5.

*4-Methoxybenzyl 4-benzamido-3-oxobutanoate (**16**)*, yellow liquid (50%). ¹H NMR (400 MHz, CDCl₃) *δ* 3.73 (s, 2H), 3.75 (s, 3H), 4.20 (d, *J* = 5.6 Hz, 2H), 5.06 (s, 2H), 6.92 (d, *J*= 8.8 Hz, 2H), 7.31 (d, *J* = 8.8 Hz, 2H), 7.47-7.51 (m, 3H), 7.87 (d, *J* = 7.2 Hz, 2H), 8.86 (t, *J* = 5.6 Hz, 1H). ¹³C NMR (100 MHz, CDCl₃) *δ* 46.6, 49.3, 55.3, 66.2, 114.0, 127.5, 127.8, 128.6, 130.2, 131.7, 133.8, 159.4, 166.7, 167.1, 200.2.

*4-Methoxybenzyl 3-oxo-3-(thiophen-2-yl)propanoate (**17**),* yellow liquid (60%). ¹H NMR (400 MHz, CDCl₃) *δ* 3.83 (s, 3H), 3.97 (s, 2H), 5.15 (s, 2H), 6.89 (d, *J* = 8.8 Hz, 2H), 7.14 (t, *J =* 4.4 Hz, 1H), 7.29 (d, *J* = 8.8 Hz, 2H), 7.71 (m, 2H). ¹³C NMR (100 MHz, CDCl₃) *δ* 46.5, 55.3, 67.2, 113.9, 127.4, 128.3, 130.2, 133.2, 134.9, 143.2, 159.7, 166.9, 184.7.

*4-Methoxybenzyl 3-oxo-4-(thiophen-2-yl)butanoate (**18**),* yellow liquid (62%). ¹H NMR (400 MHz, CDCl₃) *δ* 3.54 (s, 2H), 3.83 (s, 3H), 4.02 (s, 2H), 5.12 (s, 2H), 6.88-6.93 (m, 3H), 6.98-7.00 (m, 1H), 7.25 (dd, *J =* 1.2, 5.2 Hz, 1H), 7.31 (d, *J* = 8.8 Hz, 2H). ¹³C NMR (100 MHz, CDCl₃) *δ* 43.5, 47.9, 55.3, 67.1, 114.0, 125.6, 127.2, 127.3, 127.4, 130.2, 134.1, 159.8, 166.9, 199.0.

### II- 4) General procedures for synthesis of DHPM compounds by the Biginelli reaction (Compounds 21-51 and 53-62)

*Method A*: β-ketoester (0.50 mmol), aldehyde (0.60 mmol), urea (0.75 mmol) and Yb(OTf)₃ (5 mol%) were heated at 100 °C for 45 minutes. The reaction mixture was allowed to cool down to room temperature. Ethanol (2.5 mL) was then added and the resulting mixture was left to stand at 0-4 °C for 3 days. In most cases, the precipitate that was formed was collected by filtration. When the solid did not meet the purity standard of > 95% (LC-MS), it was further chromatographied (SiO₂). In a few cases no precipitate was formed. Solvents and volatiles were then evaporated under reduced pressure and the resulting residue was purified by silica-gel chromatography to afford title compound.

*Method B*: β-ketoester (0.50 mmol), aldehyde (0.60 mmol), urea (0.75 mmol) and Zn(OTf)₂ (10 mol%) were dissolved in acetonitrile and refluxed for 2-16 h. The reaction mixture was allowed to cool down to room temperature. The solvent was evaporated under reduced pressure and the residue was chromatographied on silica gel or purified by preparative HPLC to afford title compound.

*Method C*: β-ketoester (1.0 eq), aldehyde (1.0 eq), urea (2.0 eq) were dissolved in MeOH/HCl conc. = 1/1 at a final concentration of 1 mol/L (vs aldehyde). The mixture was stirred at 40 °C for 1-3 days. The reaction mixture was allowed to cool down to room temperature. The solid that was formed was collected by filtration, washed with water and/or ethanol to afford title compound.

*Preparation of 4-methoxybenzyl 6-methyl-2-oxo-4-phenyl-1,2,3,4-tetrahydropyrimidine-5-carboxylate **(1):*** Compound **1**, corresponding to the Compound ITB-9 in the publication of N. Lecat-Guillet et al., ChemBioChem, 2008, 9, 889-895, was prepared using method A from 4-methoxybenzyl acetoacetate **2** (111 mg), benzaldehyde (51 µL) and urea (45 mg). Isolation by filtration afforded 1 as a white solid (55%). mp : 162-164 °C. TLC: *R*_{f} = 0.24 (cHex/EtOAc = 1/1).

¹H NMR (400 MHz, DMSO-*d₆*) *δ* 2.25 (s, 3H), 3.74 (s, 3H), 4.95 (s, 2H), 5.13 (s, 1H), 6.86 (d, *J* = 8.8 Hz, 2H), 7.13 (d, *J*= 8.4 Hz, 2H), 7.15-7.20 (m, 2H), 7.24-7.30 (m, 3H), 7.74 (s, 1H), 9.24 (s, 1H). ¹³C NMR (100 MHz, DMSO-*d₆*) *δ* 18.3, 54.3, 55.5, 65.1, 99.4, 114.1, 126.7, 127.8, 128.8, 128.9, 130.0, 145.1, 149.4, 152.5, 159.4, 165.6. HPLC : *t*_{R} = 14.9 min. MS : *m*/*z* 353 ([M + H]⁺). HRMS-ESI-TOF (negative): *m*/*z* calcd for C₂₀H₁₉N₂O₄ 351.1345 ([M-H]⁻), found 351.1361.

*4-Methoxybenzyl 4-(3-bromophenyl)-6-methyl-2-oxo-1,2,3,4-tetrahydropyrimidine-5-carboxylate (**21**):* Method A, mp : 164-166 °C, white powder (61%), TLC : *R*_{f} = 0.23 (cHex/AcOEt 1:1)

¹H NMR (400 MHz, DMSO-*d₆*) *δ* 2.26 (s, 3H), 3.74 (s, 3H), 4.92 (d, *J*= 12.0 Hz, 1H), 4.99 (d, *J* = 12.4 Hz, 1H), 5.12 (s, 1H), 6.86 (d, *J* = 8.8 Hz, 2H), 7.13 (d, *J* = 8.8 Hz, 2H), 7.19 (d, *J =* 7.6 Hz, 2 H), 7.26-7.31 (m, 2H), 7.46 (d, *J* = 8.0 Hz, 1H), 7.78 (s, 1H), 9.32 (s, 1H). ¹³C NMR (100 MHz, DMSO-*d₆*) *δ* 18.3, 54.0, 55.5, 65.3, 98.7, 114.2, 122.0, 125.7, 128.7, 129.6, 130.0, 130.6, 131.3, 147.8, 150.0, 152.2, 159.4, 165.4. HPLC : *t*_{R} = 17.3 min. MS : *m*/*z* 431 ([M + H]⁺).

*4-Methoxybenzyl 4-(3-chlorophenyl)-6-methyl-2-oxo-1,2,3,4-tetrahydropyrimidine-5-carboxylate (**22**):* Method A, mp : 180-181 °C, white powder (56%), TLC : *R*_{f} = 0.26 (cHex/AcOEt 1:1)

¹H NMR (400 MHz, DMSO-*d₆*) *δ* 2.26 (s, 3H), 3.74 (s, 3H), 4.92 (d, *J*= 12.4 Hz, 1H), 5.00 (d, *J* = 12.0 Hz, 1H), 5.13 (d, *J* = 3.2 Hz, 1H), 6.86 (d, *J* = 8.8 Hz, 2H), 7.12-7.15 (m, 4H), 7.32-7.35 (m, 2H), 7.78 (bs, 1H), 9.30 (bs, 1H). ¹³C NMR (100 MHz, DMSO-*d₆*) *δ* 18.3, 54.0, 55.5, 65.3, 98.7, 114.1, 125.3, 126.7, 127.7, 128.7, 130.1, 131.0, 133.4, 147.5, 150.0, 152.3, 159.4, 165.4. HPLC : *t*_{R} = 16.9 min. MS : *m*/*z* 387 ([M + H]⁺).

*4-Methoxybenzyl 4-(2-fluorophenyl)-6-methyl-2-oxo-1,2,3,4-tetrahydropyrimidine-5-carboxylate (**23**):* Method A, mp : 181-183 °C, white powder (58%), TLC : *R*_{f} = 0.32 (cHex/AcOEt 4:6)

¹H NMR (400 MHz, DMSO-*d₆*) *δ* 2.27 (s, 3H), 3.73 (s, 3H), 4.89 (s, 2H), 5.43 (d, *J* = 2.8 Hz, 1H), 6.82 (d, *J* = 8.8 Hz, 2H), 7.05 (d, *J* = 8.8 Hz, 2H), 7.10-7.14 (m, 2H), 7.18-7.22 (m, 1H), 7.28-7.33 (m, 1H), 7.71 (s, 1H), 9.30 (s, 1H). ¹³C NMR (100 MHz, *DMSO-d₆*) *δ* 17.8, 48.6, 55.0, 64.6, 97.0, 113.6, 115.5 (d, *J* = 21.8 Hz), 124.5 (d, *J* = 3.2 Hz), 128.3, 128.8 (d, *J* = 3.9 Hz), 129.3, 129.4, 131.3 (d, J = 13.7 Hz), 149.6, 151.5, 158.8, 159.4 (d, *J*= 245.5 Hz), 164.8. ¹⁹F NMR (160 MHz, CFCl₃) : -119.0. HPLC : *t*_{R} = 15.5 min. MS : *m*/*z* 371 ([M + H]⁺).

*4-Methoxybenzyl 4-(3-fluorophenyl)-6-methyl-2-oxo-1,2,3,4-tetrahydropyrimidine-5-carboxylate (**24**):* Method A, mp : 173-174 °C, white powder (49%), TLC : *R*_{f} = 0.29 (cHex/AcOEt 4:6)

¹H NMR (400 MHz, DMSO-*d₆*) *δ* 2.26 (s, 3H), 3.74 (s, 3H), 4.93 (d, *J*= 12.0 Hz, 1H), 4.99 (d, *J* = 12.0 Hz, 1H), 5.15 (d, *J* = 3.2 Hz, 1H), 6.85 (d, *J* = 8.8 Hz, 2H), 6.92 (m, 1H), 7.03-7.11 (m, 2H), 7.14 (d, *J* = 8.4 Hz, 2H), 7.33-7.36 (m, 1H), 7.80 (s, 1H), 9.31 (s, 1H). ¹³C NMR (100 MHz, DMSO-*d₆*) *δ* 18.3, 53.9, 55.5, 65.2, 98.8, 113.4 (d, *J* = 21.3 Hz), 114.1, 114.5 (d, *J* = 20.9 Hz), 122.6 (d, *J* = 2.3 Hz), 128.7, 130.1, 131.0 (d, *J* = 8.0 Hz), 147.9 (d, *J* = 5.9 Hz), 150.0, 152.4, 159.4, 162.5 (d, *J* = 242.6 Hz), 165.5. ¹⁹F NMR (160 MHz, CFCl₃): - 113.1. HPLC : *t*_{R} = 15.0 min. MS : *m*/*z* 371 ([M + H]⁺).

*4-Methoxybenzyl 4-(4-fluorophenyl)-6-methyl-2-oxo-1,2,3,4-tetrahydropyrimidine-5-carboxylate (**25**):* Method A, mp : 172-175 °C, white powder (87%), TLC : *R*_{f} = 0.27 (cHex/ACOEt 1:1)

¹H NMR (400 MHz, DMSO-*d₆*) *δ* 2.25 (s, 3H), 3.74 (s, 3H), 4.93 (d, *J* = 12.0 Hz, 1H), 4.98 (d, *J* = 12.0 Hz, 1H), 5.13 (d, *J* = 3.2 Hz, 1H), 6.86 (d, *J* = 8.4 Hz, 2H), 7.09-7.14 (m, 4H), 7.18-7.22 (m, 2H), 7.76 (s, 1H), 9.28 (s, 1H). ¹³C NMR (100 MHz, DMSO-*d₆*) *δ* 18.3, 53.7, 55.5, 65.2, 99.2, 114.1, 115.1 (d, *J* = 21.2 Hz), 115.7, 128.3 (d, *J* = 8.4 Hz), 128.8, 130.0, 140.9 (d, *J* = 3.0 Hz), 149.6, 152.3, 159.4, 161.3 (d, J = 241.2 Hz), 165.5. ¹⁹F NMR (160 MHz, CFCl₃): -115.4. HPLC : *t*_{R} = 15.4 min. MS : *m*/*z* 371 ([M + H]⁺).

*4-Methoxybenzyl 4-cyclopropyl-6-methyl-2-oxo-1,2,3,4-tetrahydropyrimidine-5-carboxylate (**26**):* Method A, mp : 158-160 °C, white powder (68%), TLC : *R*_{f} = 0.36 (DCM/MeOH 95:5)

¹H NMR (400 MHz, DMSO-*d₆*) *δ* 0.12-0.28 (m, 4H), 0.89-0.94 (m, 1H), 2.17 (s, 3H), 3.63-3.66 (m, 1H), 3.75 (s, 3H), 4.99 (d, *J*= 12.0 Hz, 1H), 5.04 (d, *J* = 12.0 Hz, 1H), 6.93 (d, *J* = 8.4 Hz, 2H), 7.30-7.32 (m, 3H), 9.04 (s, 1H). ¹³C NMR (100 MHz, DMSO-*d₆*) *δ* 1.71, 2.42, 18.2, 53.1, 55.5, 65.2, 99.7, 114.2, 128.9, 130.4, 149.1, 153.4, 159.5, 166.0. HPLC : *t*_{R} = 13.2 min. MS : *m*/*z* 317 ([M + H]⁺).

*Preparation of 4-methoxybenzyl 4-(furan-2-yl)-6-methyl-2-oxo-1,2,3,4-tetrahydropyrimidine-5-carboxylate (**27**):* Compound **27** was prepared using method A from 4-methoxybenzyl acetoacetate **2** (111 mg), furan-2-carboxaldehyde (50 µL) and urea (45 mg). Isolation by chromatography on silica gel (cHex/EtOAc = 100/0 to 60/40) afforded **27** as an ochre powder (40%). mp : 158-159 °C. TLC: *R*_{f}= 0.26.

¹H NMR (400 MHz, DMSO-*d₆*) *δ* 2.23 (s, 3H), 3.74 (s, 3H), 4.99 (s, 2H), 5.20 (d, *J*= 3.2 Hz, 1H), 6.04 (d, *J* = 2.8 Hz, 1H), 6.35 (dd, *J*= 2.0, 3.2 Hz, 1H), 6.89 (d, *J* = 8.4 Hz, 2H), 7.20 (d, *J*= 8.4 Hz, 2H), 7.55 (s, 1H), 7.76 (s, 1H), 9.29 (s, 1H). ¹³C NMR (100 MHz, DMSO*-d₆*) *δ* 18.2, 48.1, 55.5, 65.2, 96.9, 105.8, 110.8, 114.2, 128.9, 129.9, 142.6, 150.4, 152.8, 156.3, 159.4, 165.5. HPLC : *t*_{R} = 13.1 min. MS : *m*/*z* 343 ([M + H]⁺). HRMS-ESI-TOF (negative): *m*/*z* calcd for C₁₈H₁₇N₂O₅ 341.1137 ([M - H]⁻), found 341.1141.

*4-Methoxybenzyl 4-(furan-3-yl)-6-methyl-2-oxo-1,2,3,4-tetrahydropyrimidine-5-carboxylate (**28**):* Method A, mp : 157-159 °C, beige solid (72%), TLC : *R*_{f} = 0.47 (cHex/AcOEt 35:65)

¹H NMR (400 MHz, DMSO-*d₆*) δ 2.21 (s, 3H), 3.74 (s, 3H), 5.03 (s, 2H), 5.08 (s, 1H), 6.30 (s, 1H), 6.90 (d, *J*= 8.4 Hz, 2H), 7.25 (d, *J* = 8.8 Hz, 2H), 7.30 (s, 1H), 7.54 (s, 1H), 7.66 (s, 1H), 9.22 (s, 1H). ¹³C NMR (100 MHz, DMSO-*d₆*) *δ* 18.2, 46.3, 55.5, 65.2, 99.1, 109.5, 114.2, 128.9, 129.4, 130.1, 139.0, 144.0, 149.6, 153.1, 159.4, 165.5. HPLC : *t*_{R} = 14.3 min. MS : *m*/*z* 343 ([M + H]⁺).

*4-Methoxybenzyl 6-methyl-2-oxo-4-(5-methylfuran-2-yl)-1,2,3,4-tetrahydropyrimidine-5-carboxylate (**29**):* Method A, mp : 157-159 °C, ochre powder (52%), TLC : *R*_{f} = 0.29 (cHex/AcOEt 1:1)

¹H NMR (400 MHz, DMSO-*d₆*) *δ* 2.20 (s, 3H), 2.23 (s, 3H), 3.74 (s, 3H), 4.97 (d, *J* = 12.0 Hz, 1H), 5.01 (d, *J* = 12.4 Hz, 1H), 5.13 (d, *J* = 3.2 Hz, 1H), 5.88 (d, *J* = 3.2 Hz, 1H), 5.94 (m, 1H), 6.89 (d, *J* = 8.4 Hz, 2H), 7.21 (d, *J* = 8.8 Hz, 2H), 7.72 (s, 1H), 7.76 (s, 1H), 9.24 (s, 1H). ¹³C NMR (100 MHz, DMSO-*d₆*) *δ* 13.8, 18.2, 48.2, 55.5, 65.1, 97.1, 106.5, 106.8, 114.2, 129.0, 129.9, 150.2, 151.1, 152.7, 154.7, 159.4, 165.3. HPLC : *t*_{R} = 12.9 min. MS : *m*/*z* 357 ([M + H]⁺).

*4-Methoxybenzyl 4-(thiophen-2-yl)-6-methyl-2-oxo-1,2,3,4-tetrahydropyrimidine-5-carboxylate (**30**):* Method A, mp : 152-154 °C, ochre powder (59%), TLC : *R*_{f} = 0.32 (cHex/AcOEt 1:1)

¹H NMR(400 MHz, DMSO-*d₆*) *δ* 2.23 (s, 3H), 3.74 (s, 3H), 5.02 (s, 2H), 5.40 (d, *J* = 3.6 Hz, 1H), 6.84(d,*J* = 3.2 Hz, 1H), 6.89 (d, *J* = 6.4 Hz, 2H), 6.91-6.94 (m, 1H), 7.22 (d, *J* = 8.4 Hz, 2H), 7.35 (d, *J* = 4.4 Hz, 1H), 7.90 (s, 1H), 9.35 (s, 1H). ¹³C NMR (100 MHz, DMSO-*d₆*) *δ* 18.2, 49.7, 55.5, 65.3, 99.9, 114.2, 124.0, 125.1, 127.1, 128.8, 130.0, 149.2, 149.7, 152.6, 159.4, 165.3. HPLC : *t*_{R} = 14.6 min. MS : *m*/*z* 359 ([M + H]⁺).

*4-Methoxybenzyl 6-methyl-2-oxo-4-(thiophen-3-yl)-1,2,3,4-tetrahydropyrimidine-5-carboxylate (**31**):* Method A, mp : 156-158 °C, ochre powder (70%), TLC : *R*_{f} = 0.26 (cHex/AcOEt 1:1)

¹H NMR (400 MHz, DMSO-*d₆*) *δ* 2.22 (s, 3H), 3.74 (s, 3H), 4.99 (d, *J* = 12.0 Hz, 1H), 5.03 (d, *J* = 12.4 Hz, 1H), 5.20 (d, *J* = 3.6 Hz, 1H), 6.88 (d, *J* = 8.4 Hz, 2H), 6.95 (d, *J* = 4.8 Hz, 1H), 7.09 (d, *J* = 2.4 Hz, 1H), 7.20 (d, *J* = 8.4 Hz, 2H), 7.45 (dd, J = 2.8, 4.8 Hz, 1H), 7.76 (s, 1H), 9.23 (s, 1H). ¹³C NMR (100 MHz, DMSO-*d₆*) *δ* 18.2, 49.8, 55.5, 65.2, 99.6, 114.2, 121.3, 126.6, 127.1, 128.9, 130.0, 146.1, 149.5, 153.0, 159.4, 165.6. HPLC : *t*_{R} = 14.3 min. MS : *m*/*z* 359 ([M + H]⁺).

*Benzyl 6-methyl-2-oxo-4 phenyl-1,2,3,4-tetrahydropyrimidine-5-carboxylate (32):* Method C, mp : 174-175 °C, white solid (88%), TLC : *R*_{f}= 0.35 (cHex/AcOEt 1:1)

¹H NMR (400 MHz, DMSO-*d₆*) *δ* 2.27 (s, 3H), 5.00 (d, *J* = 12.8 Hz, 1H), 5.06 (d, *J* = 12.8 Hz, 1H), 5.17 (d, *J* = 2.8 Hz, 1H), 7.14-7.32 (m, 10H), 7.77 (s, 1H), 9.28 (s, 1H). ¹³C NMR (100 MHz, DMSO-*d₆*) *δ* 18.3, 54.4, 65.3, 99.2, 126.7, 127.8, 128.0, 128.2, 128.7, 128.9, 137.0, 145.1, 149.7, 152.4, 165.5. HPLC : *t*_{R} = 15.2 min. MS : *m*/*z* 323 ([M + H]⁺).

*2-Chlorobenzyl 6-methyl-2-oxo-4-phenyl-1,2,3,4-tetrahydropyrimidine-5-carboxylate (**33**):* Method A, mp : 193-195 °C, white powder (73%), TLC : *R*_{f}= 0.64 (cHex/AcOEt 25:75)

¹H NMR (400 MHz, DMSO-*d₆*) *δ* 2.27 (s, 3H), 5.05-5.17 (m, 3H), 7.10-7.13 (m, 1H), 7.19-7.31 (m, 7H), 7.45 (d, *J* = 7.6 Hz, 1H), 7.79 (s, 1H), 9.32 (s, 1H). ¹³C NMR (100 MHz, DMSO-*d₆*) *δ* 18.3, 54.4, 62.7, 98.9, 126.7, 127.6, 127.8, 128.9, 129.7, 130.2, 130.3, 132.8, 134.3, 145.0, 150.2, 152.4, 165.3. HPLC : *t*_{R} = 17.7 min. MS : *m*/*z* 357 ([M + H]⁺).

*4-Chlorobenzyl 6-methyl-2-oxo-4-phenyl-1,2,3,4-tetrahydropyrimidine-5-carboxylate (**34**):* Method A, mp : 216-217 °C, white powder (58%), TLC : *R*_{f}= 0.49 (cHex/AcOEt 25:75)

¹H NMR (400 MHz, DMSO-*d₆*) *δ* 2.27 (s, 3H), 4.97 (d, *J* = 12.8 Hz, 1H), 5.02 (d, *J* = 13.2 Hz, 1H), 5.17 (d, *J* = 3.2 Hz, 1H), 7.14-7.35 (m, 9H), 7.78 (s, 1H), 9.30 (s, 1H). ¹³C NMR (100 MHz, DMSO-*d₆*) *δ* 18.3, 54.4, 64.4, 99.0, 126.8, 127.8, 128.7, 128.9, 129.9, 132.7, 136.0, 145.1, 150.0, 152.3, 165.4. HPLC : *t*_{R} = 17.4 min. MS : *m*/*z* 357 ([M + H]⁺).

*4-Fluorobenzyl 6-methyl-2-oxo-4-phenyl-1,2,3,4-tetrahydropyrimidine-5-carboxylate (**35**):* Method A, mp : 196-197 °C, white powder (66%), TLC : *R*_{f}= 0.60 (cHex/AcOEt 25:75)

¹H NMR (400 MHz, DMSO-*d₆*) *δ* 2.27 (s, 3H), 4.97 (d, *J* = 12.8 Hz, 1H), 5.04 (d, *J* = 12.8 Hz, 1H), 5.16 (d, *J* = 3.2 Hz, 1H), 7.09-7.32 (m, 9H), 7.75 (s, 1H), 9.27 (s, 1H). ¹³C NMR (100 MHz, DMSO-*d₆*) *δ* 18.3, 54.4, 64.6, 99.1, 115.5 (d, *J* = 21.2 Hz), 126.7, 127.8, 128.9, 130.3 (d, *J* = 8.3 Hz), 133.2 (d, J = 3.0 Hz), 145.1, 149.8, 152.4, 162.1 (d, *J* = 242.3 Hz), 165.5. ¹⁹F NMR (160 MHz, CFCl₃) : -114.5. HPLC : *t_{R}* = 15.6 min. MS : *m*/*z* 341 ([M + H]⁺).

*2-Methylbenzyl 6-methyl-2-oxo-4-phenyl-1,2,3,4-tetrahydropyrimidine-5-carboxylate (**36**)*: Method A, mp : 157-159 °C, white powder (54%), TLC : *R*_{f} = 0.69 (cHex/AcOEt 25:75)

¹H NMR (400 MHz, DMSO-*d₆*) *δ* 2.12 (s, 3H), 2.27 (s, 3H), 4.99 (d, *J* = 12.8 Hz, 1H), 5.06 (d, *J* = 12.8 Hz, 1H), 5.14 (d, *J* = 3.2 Hz, 1H), 7.06-7.26 (m, 9H), 7.73 (s, 1H), 9.25 (s, 1H). ¹³C NMR (100 MHz, DMSO-*d₆*) δ 18.2, 18.7, 54.3, 63.7, 99.1, 126.1, 126.7, 127.8, 128.4, 128.9, 129.0, 130.4, 134.8, 136.7, 145.0, 149.8, 152.4, 165.5. HPLC : *t_{R}* = 16.8 min. MS : *m*/*z* 337 ([M + H]⁺).

*3-Methoxybenzyl 6-methyl-2-oxo-4-phenyl-1,2,3,4-tetrahydropyrimidine-5-carboxylate (37):* Method A, mp : 73-75 °C, white powder (63%), TLC : *R_{f}* = 0.35 (cHex/AcOEt 1:1).

¹H NMR (400 MHz, DMSO-*d*₆) *δ* 2.28 (s, 3H), 3.69 (s, 3H), 5.00 (s, 1H), 5.01 (s, 1H), 5.17 (d, *J=* 2.8 Hz, 1H), 6.72 (d, *J* = 7.6 Hz, 1H), 6.77 (s, 1H), 6.84 (d, *J* = 8.4 Hz, 1H), 7.18-7.30 (m, 6H), 7.77 (s, 1H), 9.28 (s, 1H). ¹³C NMR (100 MHz, DMSO-*d₆*) *δ* 18.3, 54.3, 55.4, 65.2, 99.1, 113.4, 113.7, 120.0, 126.7, 127.8, 128.9, 129.9, 138.5, 145.0, 149.8, 152.5, 159.6, 165.5. HPLC: *t*_{R}= 15.1 min. MS : *m*/*z* 394 ([M + H + CH₃CN]⁺).

*Benzo[d][1,3]dioxol-5-ylmethyl 6-methyl-2-oxo-4-phenyl-1,2,3,4-tetrahydropyrimidine-5-carboxylate (38):* Method A, mp : 186-187 °C, white powder (59%), TLC : *R*_{f} = 0.54 (cHex/AcOEt 25:75)

¹H NMR (400 MHz, DMSO-*d₆*) *δ* 2.26 (s, 3H), 4.89 (d, *J=* 12.0 Hz, 1H), 4.94 (d, *J=* 12.4 Hz, 1H), 5.18 (d, *J =* 3.2 Hz, 1H), 6.00 (s, 2H), 6.68-6.71 (m, 2H), 6.82 (d, *J =* 7.6 Hz, 1H), 7.19 (d, *J =* 7.6 Hz, 2H), 7.24-7.28 (m, 3H), 7.74 (s, 1H), 9.24 (s, 1H). ¹³C NMR (100 MHz, DMSO-*d₆*) *δ* 18.3, 54.3, 65.3, 99.3, 101.4, 108.4, 108.9, 122.1, 126.7, 127.8, 128.8, 130.6, 145.1, 147.3, 147.6, 149.6, 152.5, 165.5. HPLC *: t*_{R}*, =* 14.4 min. MS : *m*/*z* 367 ([M + H]⁺).

*Allyl 6-methyl-4-phenyl-2-thioxo-1,2,3,4-tetrahydropyrimidine-5-carboxylate (39):* Method C, mp : 147-149°C, yellow solid (52%), TLC : *R*_{f}= 0.82 (cHex/AcOEt 4:6)

¹H NMR (400 MHz, DMSO-*d₆*) *δ* 2.32 (s, 3H), 4.47-4.57 (m, 2H), 5.07-5.10 (m, 1H), 5.11-5.13 (m, 1H), 5.20 (d, *J = 3.6 Hz,* 1H), 5.80-5.87 (m, 1H), 7.22-7.37 (m, 5H), 9.69 (s, 1H), 10.40 (s, 1H). ¹³C NMR (100 MHz, DMSO-*d₆*) *δ* 17.2, 53.9, 64.0, 100.2, 117.2, 126.4, 127.7, 128.6, 132.7,143.3, 145.7, 164.7, 174.2. MS *m*/*z* 289 ([M + H]⁺).

*4-Methoxybenzyl 6-ethyl-2-oxo-4-phenyl-1,2,3,4-tetrahydropyrimidine-5-carboxylate **(40):*** Method A, mp : 62-65 °C, white powder (66%), TLC : R_{f} = 0.26 (cHex/AcOEt 1:1)

¹H NMR (400 MHz, DMSO-*d₆*) *δ* 1.09 (t, *J* = 7.4 Hz, 3H), 2.56-2.62 (m, 1H), 2.68-2.74 (m, 1H), 3.74 (s, 3H), 4.96 (s, 2H), 5.12 (d, *J* = 3.6 Hz, 1H), 6.86 (d, *J =* 8.4 Hz, 2H), 7.13 (d, *J =* 8.4 Hz, 2H), 7.17-7.30 (m, 5H), 7.72 (s, 1H), 9.23 (s, 1H). ¹³C NMR (400 MHz, DMSO-*d₆*) *δ* 13.5, 24.5, 54.3, 55.5, 65.2, 98.5, 114.1, 126.7, 127.7, 128.7, 128.9, 130.0, 145.1, 152.7, 154.9, 159.4, 165.2. *HPLC : t_{R} =* 16.4 min. MS : *m*/*z* 367 ([M + H]⁺).

*4-Methoxybenzyl 2-oxo-6-isobutyl-4-phenyl-1, 2,3,4-tetrahydropyrimidine-5-carboxylate **(41):*** Method A, mp : 190-192 °C, white powder (23%), TLC : *R*_{f} = 0.34 (cHex/AcOEt 1:1)

¹H NMR (400 MHz, DMSO-*d₆*) *δ* 0.81 (d, *J =* 2.0 Hz, 3H), 0.82 (d, *J =* 2.4 Hz, 3H), 1.87-1.93 (m, 1H), 2.47-2.60 (m, 2H), 3.74 (s, 3H), 4.93 (d, *J =* 12.0 Hz, 1H), 4.97 (d, *J =* 12.0 Hz, 1H), 5.16 (d, *J =* 3.6 Hz, 1H), 6.87 (d, *J =* 8.8 Hz, 2H), 7.16-7.28 (m, 7H), 7.73 (s, 1H), 9.14 (s, 1H). ¹³C NMR (400 MHz, DMSO-*d₆*) *δ* 22.3, 22.5, 28.3, 39.1, 54.4, 55.5, 65.4, 100.1, 114.1, 126.6, 127.7, 128.6, 128.8, 130.3, 145.2, 152.4, 152.7, 159.5, 165.5. HPLC: t*_{R}* =19.4 min. MS : *m*/*z* 423 ([M + H]⁺).

*4-Methoxybenzyl 2-oxo-6-pentyl-4-phenyl-1,2,3,4-tetrahydropyrimidine-5-carboxylate **(42):*** Method A, mp : 54-56 °C, white powder (77%), TLC : *R*_{f}= 0.55 (cHex/AcOEt 4:6)

¹H NMR (400 MHz, DMSO-*d₆*) *δ* 0.82 (t, *J=* 7.0 Hz, 3H), 1.10-1.25 (m, 4H), 1.44-1.48 (m, 2H), 2.52-2.55 (m, 2H), 3.74 (s, 3H) , 4.93 (d, *J=* 12.0 Hz, 1H), 4.97 (d, *J=* 12.0 Hz, 1H), 5.12 (d, *J =* 3.2 Hz, 1H), 6.87 (d, *J* = 8.4 Hz, 2H), 7.15 -7.18 (m, 4H), 7.24-7.32 (m, 3H), 7.72 (s, 1H), 9.19 (s, 1H). ¹³C NMR (100 MHz, DMSO-*d₆*) *δ* 14.3, 22.3, 31.1, 31.5, 54.3, 55.5, 65.4, 99.2, 114.1, 126.6, 127.8, 128.6, 128.9, 130.2, 145.0, 152.8, 153.5, 159.4, 165.5. HPLC : *t*_{R} = 21.5 min. MS : *m*/*z* 450 ([M + H + CH₃CN]⁺).

*4-Methoxybenzyl 6-cyclohexyl-2-oxo-4-phenyl-1, 2,3,4-tetrahydropyrimidine-5-carboxylate **(43):*** Method A, mp : 98-101 °C, white powder (54%), TLC : *R*_{f} = 0.42 (cHex/AcOEt 1:1)

¹H NMR (400 MHz, DMSO-*d₆*) *δ* 1.15-1.24 (m, 4H), 1.49-1.71 (m, 7H), 3.74 (s, 3H), 5.12 (d, *J =* 3.6 Hz, 1H), 6.86 (d, *J=* 8.8 Hz, 2H), 7.12-7.16 (m, 4H), 7.22-7.30 (m, 3H), 7.71 (s, 1H), 8.83 (s, 1H). ¹³C NMR (100 MHz, DMSO-*d₆*) *δ* 25.3, 26.2, 26.4, 28.7, 29.0, 38.1, 54.2, 55.5, 65.4, 98.5, 114.1, 126.6, 127.8, 128.6, 128.9, 130.1, 144.9, 152.9, 156.6, 159.4, 165.7. HPLC : *t*_{R} = 21.7 min. MS : *m*/*z* 462 ([M + H + CH₃CN]⁺). *4-Methoxybenzyl 4,6-diphenyl-2-oxo-1,2,3,4-tetrahydropyrimidine-5-carboxylate **(44):*** Method A, mp: 82-84 °C, white powder (44%), TLC : *R*_{f}= 0.33 (cHex/AcOEt 4:6) ¹H NMR (400 MHz, DMSO-*d₆*) *δ* 3.72 (s, 3H), 4.70 (d, *J =* 12.4 Hz, 1H), 4.75 (d, *J =* 12.4 Hz, 1H), 5.23 (d, *J* = 3.2 Hz, 1H), 6.75 (d, *J =* 8.8 Hz, 2H), 6.80 (d, *J* = 8.4 Hz, 2H), 7.29-7.31 (m, 3H), 7.33-7.37 (m, 7H), 7.88 (s, 1H), 9.33 (s, 1H). ¹³C NMR (100 MHz, DMSO-*d₆*) *δ* 54.6, 55.5, 65.2, 100.4, 113.9, 126.7, 127.9, 128.2, 128.3, 128.8, 129.0, 129.4, 129.7, 135.3, 144.7, 149.9, 152.5, 159.2, 165.3. HPLC : *t*_{R} = 1.7.8 min. MS : *m*/*z* 456 ([M + H + CH₃CN]⁺).

*4-Methoxybenzyl 6-benzyl-2-oxo-4 phenyl-1,2,3,4-tetrahydropyrimidine-5-carboxylate **(45):*** Method A, mp : 140-142 °C, white powder (64%), TLC : *R*_{f} = 0.23 (cHex/AcOEt 1:1)

¹HNMR (400 MHz, DMSO-*d₆*) *δ* 3.74 (s, 3H), 3.92 (d,*J*= 13.6 Hz, 1H), 4.19 (d, *J =* 14.0 Hz, 1H), 4.96 (s, 2H), 5.18 (d, *J =* 3.6 Hz, 1H), 6.84 (d, *J =* 8.4 Hz, 2H), 7.09 (d, *J =* 8.4 Hz, 2H), 7.16-7.19 (m, 2H), 7.21-7.31 (m, 8H), 7.78 (s, 1H), 9.36 (s, 1H). ¹³C NMR (400 MHz, DMSO-*d₆*) *δ* 35.8, 54.3, 55.5, 65.4, 100.3, 114.1, 126.7, 126.9, 127.8, 128.5, 128.7, 128.8, 128.9, 130.1, 138.1, 144.9, 150.9, 152.6, 159.4, 165.4. HPLC : *t*_{R} = 19.8 min. MS: *m*/*z* 429 ([M + H]⁺).

*4-Methoxybenzyl 2-oxo-6-phenoxymethyl-4-phenyl-1,2,3,4-tetrahydropyrimidine-5-carboxylate **(46):*** Method A, mp : 171-173 °C, white powder (23%), TLC : *R*_{f} = 0.27 (cHex/AcOEt 1:1)

¹H NMR (400 MHz, DMSO-*d₆*) *δ* 3.73 (s, 3H), 4.96-5.23 (m, 5H), 6.81 (d, *J=* 8.8 Hz, 2H), 6.90-6.95 (m, 3H), 7.12 (d, *J =* 8.8 Hz, 2H), 7.21-7.29 (m, 7H), 7.86 (s, 1H), 9.23 (s, 1H). ¹³C NMR (100 MHz, DMSO-*d₆*) *δ* 54.4, 55.5, 64.0, 65.8, 101.5, 114.1, 115.1, 121.6, 126.8, 128.0, 128.3, 129.0, 129.9, 130.2, 144.3, 146.6, 152.3, 158.2, 159.4, 165.0. HPLC : *t*_{R} =21.1 min. MS : *m*/*z* 486 ([M + H + CH₃CN]⁺).

*4-Methoxybenzyl 2-oxo-6-(benzamidomethyl)-4-phenyl-1,2,3,4-tetrahydropyrimidine-5-carboxylate **(47):*** Method A, mp : 213-216 °C, white powder (42%), TLC : *R*_{f} = 0.15 (cHex/AcOEt 1:1)

¹H NMR (400 MHz, DMSO-*d₆*) *δ* 3.73 (s, 3H), 4.54 (dd, *J=* 5.6, 15.6 Hz, 1H), 4.77 (dd, *J =* 5.6, 15.6 Hz, 1H), 5.00 (s, 2H), 5.18 (d, *J =* 3.2 Hz, 1H), 6.84 (d, *J =* 8.8 Hz, 2H), 7.14 (d, *J=* 8.8 Hz, 2H), 7.22-7.30 (m, 5H), 7.48-7.57 (m, 3H), 7.82 (s, 1H), 7.88 (d, *J=* 8.4 Hz, 2H), 8.69 (t, *J=* 5.6 Hz, 1H), 8.82 (s, 1H). ¹³C NMR (100 MHz, DMSO-*d₆*) *δ* 31.2,54.5, 55.5, 65.5, 100.1, 114.1, 126.8, 127.9, 128.6, 128.8, 128.9, 130.0, 132.0, 134.1, 144.6, 149.2, 152.2, 159.4, 165.2. HPLC: *t*_{R} = 17.2 min. MS : *m*/*z* 472 ([M + H]⁺).

*4-Methoxybenzyl 2-oxo-4-phenyl-6-(thiophen-2-yl)-1,2,3,4-tetrahydropyrimidine-5-carboxylate **(48):*** Method A, mp : 78-80 °C, yellow powder (10%), TLC : R_{f} = 0.20 (cHex/AcOEt 1: 1)

¹H NMR (400 MHz, DMSO-*d₆*) *δ* 3.73 (s, 3H), 4.80 (d, J = 12.4 Hz, 1H), 4.85 (d, *J=* 12.0 Hz, 1H), 5.20 (d, *J =* 3.6 Hz, 1H), 6.81 (d, *J =* 8.8 Hz, 2H), 6.95 (d, *J =* 8.4 Hz, 2H), 7.05-7.07 (m, 1H), 7.23-7.25 (m, 1H), 7.29-7.38 (m, 5H), 7.67 (dd, *J =* 0.8, 5.2 Hz, 1H), 7.92 (s, 1H), 9.38 (s, 1H). ¹³C NMR (100 MHz, DMSO-*d₆*) *δ* 54.6, 55.5, 65.5, 102.4, 114.0, 126.6, 127.3, 128.0, 128.2, 128.7, 129.0, 129.8, 129.9, 134.7, 142.0, 144.2, 152.5, 159.3, 165.3. HPLC*: t*_{R} *=* 17.3 min. MS : *m*/*z* 421 ([M + H]⁺).

*4-Methoxybenzyl 2-oxo-4-phenyl-6-(thiophen-2-ylmethyl)-1,2,3,4-tetrahydropyrimidine-5-carboxylate **(49):*** Method A, mp : 140-142 °C, white powder (14%), TLC : *R*_{f}*=* 0.30 (cHex/AcOEt 1: 1)

¹H NMR (400 MHz, DMSO-*d₆*) *δ* 3.74 (s, 3H), 4.12 (d, *J=* 14.0 Hz, 1H), 4.31 (d, *J=* 13.6 Hz, 1H), 5.00 (s, 2H), 5.16 (d, *J* = 3.2 Hz, 1H), 6.84 (d, *J* = 8.4 Hz, 2H), 6.93 (dd, *J* = 3.6, 5.2 Hz, 1H), 6.99 (d, *J =* 2.8 Hz, 1H), 7.11-7.17 (m, 4H), 7.24-7.27 (m, 3H), 7.34 (dd, *J =* 0.8, 5.2 Hz, 1H), 7.78 (s, 1H), 9.44 (s, 1H). ¹³C NMR (400 MHz, DMSO-*d₆*) *δ* 30.8, 54.3, 55.5, 65.5, 99.6, 114.1, 125.3, 126.7, 126.7, 126.9, 127.9, 128.5, 128.9, 130.1, 139.9, 144.7, 150.5, 152.5, 159.4,165.2 . HPLC: *t*_{R} = 19.1 min. MS : *m*/*z* 476 ([M + H + CH₃CN]⁺).

*4-Methoxybenzyl 1, 6-dimethyl-2-oxo-4-phenyl-1,2,3,4-tetrahydropyrimidine-5-carboxylate **(50):*** Method B, purified by flash chromatography (SiO₂, cHex/AcOEt), mp: 134-136 °C, yellow solid (71%), TLC : *R*_{f}= 0.43 (cHex/AcOEt 4:6)

¹H NMR (400 MHz, DMSO-*d₆*) *δ* 2.50 (s, 3H), 3.10 (s, 3H), 3.74 (s, 3H), 5.00 (s, 2H), 5.14 (d, *J =* 4.0 Hz, 1H), 6.87 (d, *J =* 8.8 Hz, 2H), 7.15-7.17 (m, 4H), 7.24-7.30 (m, 3H), 7.96 (d, *J* = 4.0 Hz, 1H). ¹³C NMR (100 MHz, DMSO-*d₆*) *δ* 16.5, 30.2, 52.7, 55.5, 65.5, 102.6, 114.2, 126.5, 127.8, 128.7, 128.9, 130.1, 144.3, 146.6, 151.6, 159.4, 165.8. HPLC *: t_{R} =* 17.6 min. MS : *m*/*z* 367 ([M + H]⁺).

*4-Metboxybenzyl 1,3,6-trimethyl-*2*-oxo-4-phenyl-1,2,3,4-tetrahydropyrimidine-5-carboxylate **(51):*** Method B, purified by flash chromatography (SiO₂, cHex/AcOEt), yellow liquid (35%)

¹HNMR (400 MHz, DMSO-*d₆*) *δ* 2.51 (s, 3H), 2.90 (s, 3H), 3.28 (s, 3H), 3.84 (s, 3H), 5.04 (d, *J =* 12.4 Hz, 1H), 5.10 (d, *J =* 12.0 Hz, 1H), 5.23 (s, 1H), 6.88 (d, *J =* 8.4 Hz, 2H), 7.14-7.27 (m, 7H). ¹³C NMR (100 MHz, DMSO-*d₆*) *δ* 16.7, 31.0, 34.4, 55.3, 60.8, 65.9, 103.3, 113.8, 126.7, 127.8, 128.2, 128.6, 130.1, 140.8, 149.7, 153.7, 159.5, 165.8. HPLC : *t*_{R} = 20.8 min. MS : *m*/*z* 381 ([M + H]⁺).

*4-Methoxybenzyl 4-(furan-2-yl)-1,6-dimethyl-2-oxo-1,2,3,4-tetrahydropyrimidine-5-carboxylate **(53):*** Method B, yellow solid (49 %), mp : 112-113 °C. TLC : *R*_{f} = 0.28 (cHex/AcOEt 1:1)

¹H NMR (400 MHz, CDCl₃) *δ* 2.49 (s, 3H), 3.15 (s, 3H), 3.76 (s, 3H), 5.03 (s, 1H), 5.04 (s, 1H), 5.41 (d, *J*= 3.6 Hz, 1H), 5.97 (d, *J =* 3.2 Hz, 1H), 6.15-6.19 (m, 1H), 6.32 (d, *J =* 3.2 Hz, 1H), 6.82 (d, *J=* 8.8 Hz, 2H), 7.16 (d, *J =* 8.8 Hz, 2H), 7.24 (d, *J =* 0.8 Hz, 1H). ¹³C NMR (100 MHz, CDCl₃) *δ* 16.6, 30.4, 47.6, 55.3, 65.9, 101.3, 105.8, 110.2, 113.9, 128.3, 129.8, 142.3, 151.4, 154.5, 155.0, 159.5, 165.6. HPLC : *t*_{R} = 16.1 min. MS *m*/*z* 357 ([M + H]⁺).

*Preparation of 4-methoxybenzyl 4-(furan-2-yl)-1,3,6-trimethyl-2-oxo-1,2,3,4-tetrahydropyrimidine-5-carboxylate **(54):*** Compound **54** was prepared using method B from 4-methoxybenzyl 3-oxobutanoate **2** (111 mg), furan-2-carboxaldehyde (50 µL) and *N,N'-*dimethylurea (66 mg). Isolation by chromatography on silica gel (cHex/EtOAc = 100/0 to 70/30) afforded **54** as a yellow oil (46%). TLC: *R*_{f}=0.28 (cHex/EtOAc =1/1).

¹H NMR (400 MHz, CDCl₃) *δ* 2.48 (s, 3H), 2.96 (s, 3H), 3.20 (s, 3H), 3.78 (s, 3H), 5.02 (d, *J =* 12.4 Hz, 1H), 5.08 (d, *J =* 12.0 Hz, 1H), 5.28 (s, 1H), 6.00 (d, *J* = 2.8 Hz, 1H), 6.22-6.23 (m, 1H), 6.84 (d, *J =* 8.8 Hz, 2H), 7.18 (d, *J =* 8.8 Hz, 2H), 7.26 (s, J = 0.8 Hz, 1H). ¹³C NMR (100 MHz, CDCl₃) *δ* 16.7, 31.3, 34.9, 54.5, 55.4, 66.0, 100.5, 107.0, 110.2, 114.0, 128.5, 129.9, 142.5, 151.5, 153.3, 154.1, 159.6, 165.6. HPLC : *t*_{R} = 19.1 min. MS : *m*/*z* 371 ([M + H]⁺). HRMS : calculated 371.1607 found 371.1592 ([M + H]⁺).

*Preparation of 3-methoxybenzyl 4-(furan-2-yl)-6-methyl-2-oxo-1,2,3,4-tetrahydropyrimidine-5-carboxylate **(55):*** Compound **55** was prepared using method B from 3-methoxybenzyl 3-oxobutanoate **7** (111 mg), furan-2-carboxaldehyde (50 µL) and urea (45 mg). Isolation by chromatography on silica gel (cHex/EtOAc = 100/0 to 50/50) afforded **55** as an orange solid (70%). mp : 165-166 °C. TLC: R_{f} *=* 0.35 (cHex/EtOAc = 4/6).

¹H NMR (400 MHz, CDCl₃) *δ* 2.38 (s, 3H), 3.78 (s, 3H), 5.07 (d, *J=* 12.8 Hz, 1H), 5.13 (d, *J* = 12.8 Hz, 1H), 5.52 (m, 2H), 6.09 (d, *J =* 3.2 Hz, 1H), 6.25-6.27 (m, 1H), 6.78-6.85 (s, 3H), 7.21-7.32 (m, 3H). ¹³C NMR (100 MHz, CDCl₃) *δ* 19.1, 49.1, 55.4, 66.1, 89.1, 106.4, 110.5, 113.5, 113.7, 120.3, 129.8, 137.8, 142.7, 154.7, 157.1, 159.9, 171.8. HPLC : *t*_{R} = 13.4 min. MS : *m*/*z* 343 ([M + H]⁺). HRMS: calculated 341.1137 found 341.1140 ([M - H]⁻).

*3-Methoxybenzyl 4-(furan-2-yl)-1,6-dimethyl-2-oxo-1,2,3,4-tetrahydropyrimidine-5-carboxylate **(56):*** Method B, yellow solid (51 %), mp : 155-156 °C. TLC : *R*_{f} = 0.27 (cHex/AcOEt 1:1)

¹H NMR (400 MHz, CDCl₃) *δ* 2.51 (s, 3H), 3.18 (s, 3H), 3.74 (s, 3H), 5.06 (d, *J=* 8.4 Hz, 1H), 5.12 (d, *J =* 8.4 Hz, 1H), 5.45 (d, *J =* 2.8 Hz, 1H), 6.01-6.02 (d, *J* = 2.8 Hz, 1H), 6.07 (d, *J=* 2.8 Hz, 1H), 6.20-6.22 (m, 1H), 6.77-6.82 (m, 3H), 7.19-7.23 (m, 1H), 7.26 (s, 1H). ¹³C NMR (100 MHz, CDCl₃) *δ* 16.7, 30.5, 47.6, 55.3, 66.0, 101.2, 105.9, 110.3,113.4,113.7, 120.2, 129.6, 137.8, 142.4, 151.8, 154.5, 154.9, 159.8, 165.5. HPLC : *t*_{R} = 16.3 min. MS *m*/*z* 357 ([M + H]⁺).

*Preparation of 3-methoxybenzyl 4-(furan-2yl)-1,3,6-trimethyl-2-oxo-1,2,3,4-tetrahydropyrimidine-5-carboxylate **(57):*** Compound **57** was prepared using method B from 3-methoxybenzyl 3-oxobutanoate **7** (111 mg), furan-2-carboxaldehyde (50 µL) and *N,N'-*dimethylurea (66 mg). Isolation by chromatography on silica gel (cHex/EtOAc = 100/0 to 70/30) afforded **57** as a yellow oil (43%). TLC: *R*_{f} *=* 0.31 (cHex/EtOAc = 6/4).

¹H NMR (400 MHz, CDCl₃) *δ* 2.50 (s, 3H), 2.97 (s, 3H), 3.21 (s, 3H), 3.75 (s, 3H), 5.05 (d, *J* =12.4 Hz, 1H), 5.13 (d, *J =* 12.8 Hz, 1H), 5.32 (s, 1H), 6.03 (d, *J =* 3.2 Hz, 1H), 6.21-6.23 (m, 1H), 6.79-6.82 (m, 3H), 7.20-7.24 (m, 1H), 7.27 (s, *J* = 0.8 Hz, 1H). ¹³C NMR (100 MHz, CDCl₃) *δ* 16.7, 31.3, 34.9, 54.4, 55.3, 66.0, 100.3, 107.0, 110.2, 113.4, 113.7, 120.2, 129.7, 137.9, 142.5, 151.8, 153.3, 154.1, 159.9, 165.4. HPLC : *t*_{R} = 19.3 min. MS : *m*/*z* 371 ([M + H]⁺). HRMS : calculated 371.1607 found 371.1595 ([M + H]⁺).

*3,4-Dimethoxybenzyl 4-(furan-2-yl)-1,3,6-trimethyl-2-oxo-1,2,3,4-tetrahydropyrimidine-5-carboxylate **(58):*** Method B, white solid (44 %), mp : 122-124 °C. TLC : *R*_{f}= 0.28 (cHex/AcOEt 6:4)

¹H NMR (400 MHz, CDCl₃) *δ* 2.50 (s, 3H), 2.98 (s, 3H), 3.21 (s, 3H), 3.71 (s, 3H), 3.74 (s, 3H), 5.11 (d, *J =* 13.2 Hz, 1H), 5.19 (d, *J =* 12.8 Hz, 1H), 5.32 (s, 1H), 6.04 (d, *J =* 3.2 Hz, 1H), 6.21-6.22 (m, 1H), 6.77-6.78 (m, 3H), 7.26 (s, *J=* 0.8 Hz, 1H). ¹³CNMR (100 MHz, CDCl₃) *δ* 16.7, 30.4, 34.9, 54.5, 55.9, 56.1, 61.6, 100.7, 107.0, 110.2, 111.6 , 113.7, 115.4, 125.8, 142.5, 151.5, 151.7, 153.3, 153.6, 154.2, 165.6. HPLC : *t*_{R} = 19.5 min. MS *m*/*z* 401 ([M + H]⁺).

*Preparation of benzo[d][1,3]dioxol-5-ylmethyl 4-(furan-2-yl)-d-methyl-2-oxo-1,2,3,4-tetrahydropyrimidine-5-carboxylate **(59):*** Compound **59** was prepared using method B from 3,4-(methylenedioxy)benzyl 3-oxobutanoate **8** (118 mg), furan-2-carboxaldehyde (50 µL) and urea (45 mg). Isolation by chromatography on silica gel (cHex/EtOAc = 100/0 to 50/50) afforded **59** as a white solid (45%). mp : 169-171 °C. TLC: *R*_{f} = 0.33 (cHex/EtOAc = 4/6).

¹H NMR (400 MHz, DMSO-*d₆*) *δ* 2.24 (s, 3H), 4.95 (d, *J =* 12.0 Hz, 1H), 4.99 (d, *J =* 12.4 Hz, 1H), 5.21 (d, *J =* 3.2 Hz, 1H), 6.00 (s, 2H), 6.05 (d, *J =* 3.2Hz, 1H), 6.34-6.35 (m, 1H), 6.76 (d, *J=* 8.0 Hz, 1H), 6.81 (s, 1H), 6.86 (d, *J=* 8.0 Hz, 1H), 7.54 (d, *J=* 0.8 Hz, 1H), 7.77 (s, 1H), 9.30 (s, 1H). ¹³C NMR (100 MHz, DMSO-*d₆*) *δ* 18.2, 48.1, 65.3, 96.8, 101.4, 105.8, 108.5, 108.8, 110.8, 122.0, 130.7, 142.6, 147.3, 147.7, 150.5, 152.7, 156.3, 165.2. *HPLC : t_{R}* =12.7 min. MS : *m*/*z* 357 ([M + H]⁺). HRMS: calculated 355.0930 found 355.0930 ([M - H]⁻).

*Benzo[d][1,3]dioxol-5-ylmethyl 4-(furan-2yl)-1, 6-dimethyl-2-oxo-1, 2,3,4-tetrahydropyrimidine-5-carboxylate **(60):*** Method B, yellow solid (30 %), mp : 141-143 °C. TLC : *R*_{f} = 0.42 (cHex/AcOEt 4:6)

¹H NMR (400 MHz, DMSO-*d₆*) *δ* 2.49 (s, 3H), 3.10 (s, 3H), 5.00 (s, 2H), 5.21 (d, *J=* 4.0 Hz, 1H), 6.01 (s, 2H), 6.05 (d, *J =* 3.2 Hz, 1H), 6.34-6.35 (m, 1H), 6.78 (d, ***J =*** 8.0 Hz, 1H), 6.82 (s,1H), 6.87 (d, *J=* 8.0 Hz, 1H), 7.54 (d, *J*= 0.8 Hz, 1H), 7.96 (d, *J*= 4.0 Hz, 1H). ¹³C NMR (100 MHz, DMSO-*d₆*) *δ* 16.5, 30.3, 46.9, 65.6, 100.2, 101.5, 105.9, 108.5, 108.9, 110.8, 122.1, 130.5, 142.7, 147.4, 147.7, 152.7, 153.6, 155.9, 165.4. HPLC : *t*_{R} = 15.7 min. MS *m*/*z* 371 ([M + H]⁺).

*Preparation of benzo[d][1,3]dioxol-5-ylmethyl 4-(furan-2-yl)-1,3,6-trimethyl-2-oxo-1,2,3,4-tetrahydropyrimidine-5-carboxylate **(61):*** Compound **61** was prepared using method B from 3,4-(methylenedioxy)benzyl 3-oxobutanoate **8** (118 mg), furan-2-carboxaldehyde (50 µL) and N,N'-dimethylurea (66 mg). Isolation by chromatography on silica gel (cHex/EtOAc = 100/0 to 70/30) afforded **61** as a brown oil (70%). TLC: *R*_{f}= 0.28 (cHex/EtOAc = 1/1).

¹H NMR (400 MHz, CDCl₃) *δ* 2.51 (s, 3H), 2.99 (s, 3H), 3.23 (s, 3H), 5.00 (d, *J =* 12.0 Hz, 1H), 5.08 (d, *J =* 12.4 Hz, 1H), 5.32 (s, 1H), 5.94 (s, 2H), 6.05 (d, *J =* 3.2 Hz, 1H), 6.25-6.26 (m, 1H), 6.74-6.75 (m, 3H), 7.30 (d, *J=* 0.8 Hz, 1H). ¹³C NMR (100 MHz, CDCl₃) *δ* 16.5, 31.1, 34.7, 54.2, 65.9, 100.2, 101.1, 106.8, 108.1, 108.7, 110.1, 121.8, 130.0, 142.3, 147.4, 147.7, 151.5, 153.1, 153.9, 165.3. HPLC: *t*_{R} = 18.5min. MS: *m*/*z* 385 ([M + H]⁺). HRMS: calculated 385.1400 found 385.1386 ([M + H]⁺).

*Benzo[d][1,3]dioxol-5-ylmethyl 4-(furan-3yl)-6-methyl-2-oxo-1,2,3,4-tetrahydropyrimidine-5-carboxylate **(62):*** Method B, white solid (63 %), mp : 172-173 °C. TLC : R_{f}= 0.30 (cHex/AcOEt 4:6)

¹H NMR (400 MHz, DMSO-*d₆*) *δ* 2.21 (s, 3H), 5.00 (s, 2H), 5.10 (d, *J=* 3.2 Hz, 1H), 6.00 (s, 2H), 6.30 (d, *J =* 0.8 Hz, 1H), 6.82-6.88 (m, 3H), 7.31 (s, 1H), 7.54 (d, *J =* 1.6 Hz, 1H), 7-65 (s, 1H), 9.22 (s, 1H). ¹³C NMR (100 MHz, DMSO-*d₆*) *δ* 18.2, 46.3, 65.3, 99.0, 101.4, 108.5, 109.0, 109.5, 122.2, 129.4, 130.7, 139.0, 144.0, 147.4, 147.7, 149.8, 153.1, 165.4. HPLC : *t_{R}* = 12.5 min. MS *m*/*z* 357 ([M + H]⁺).

### II- 5) Synthetic preparation and data analysis for Compounds 19, 20, 52, 63.

*Preparation of 6-methyl-2-oxo-4-phenyl-1,2,3,4-tetrahydropyrimidine-5-carboxylic acid (19):* To a solution of **32** (21.4 mmol) in methanol (60 mL), under hydrogen atmosphere was added Pd/C 10% (0.1 eq, 2.14 mmol). The mixture was then stirred at room temperature for 3.5h. Excess solvent was removed *in vacuo* and the residue is suspended in 0.5M KOH (100 mL). After vigourous stirring for 3h at room temperature, the suspension was filtered on Celite. The filtrate was acidified to pH 1-2 with HCl 37% (~15 mL) and the resulting precipitate was collected by filtration, air-dried to afford **19** as a white solid (79%). mp : 232-233 °C. TLC: *R*_{f}= 0.35 (cHex/EtOAc = 1/1).

¹H NMR (400 MHz, DMSO-*d₆*) *δ* 2.24 (s, 3H), 5.11 (d, *J*= 3.2 Hz, 1H), 7.24-7.34 (m, 5H), 7.68 (s, 1H), 9.09 (s, 1H), 11.89 (bs, 1H). ¹³C NMR (100 MHz, DMSO-*d₆*) *δ* 18.2, 54.4, 100.3, 126.7, 127.6, 128.8, 145.3, 148.2, 152.8, 167.6. HPLC : *t*_{R} = 3.9 min. MS : *m*/*z* 233 ([M+H]⁺).

*Preparation of N-(benzo[d][1,3]dioxol-5-ylmethyl) 6-meihyl-2-oxo-4-phenyl-1,2,3,4-tetrahydropyrimidine-5-carboxamide **(63):*** In a 10 mL microwave vial, carboxylic acid **19** (200 mg), piperonylamine (107 µL), EDCI (247 mg) and HBTU (213 mg) were dissolved in dimethylacetamide (2 mL). Diisopropylethylamine (233 µL) was added and the mixture is microwaved at 80°C for 30 minutes. Excess solvent was removed *in vacuo* and the residue was disolved in ethyl acetate (20 mL). The organic solution was washed with 1.0 M HCl (2 x 5 mL) and saturated NaCl (5 mL). Organic layers were joined, dried (Na₂SO₄) and concentrated *in vacuo.* The resulting crude residue was purified by chromatography on silica gel (DCM/MeOH = 100/0 to 90/10) to afford **63** as a brown solid (36%). mp : 235-237 °C. TLC: *R*_{f} = 0.15 (cHex/EtOAc = 4/6).

¹H NMR (400 MHz, DMSO-*d₆*) *δ* 2.00 (s, 3H), 4.13 (d, *J=* 5.6 Hz, 2H), 5.29 (d, *J=* 1.6 Hz, 1H), 5.95 (s, 2H), 6.49 (d, *J =* 7.6 Hz, 1H), 6.58 (s, 1H), 6.73 (d, *J =* 7.6 Hz, 1H), 7.21-7.30 (m, 5H), 7.49 (s, 1H), 8.05 (t, *J =* 5.6 Hz, 1H), 8.55 (s, 1H). ¹³C NMR (100 MHz, DMSO-*d₆*) *δ* 17.4, 42.3, 55.4, 101.1, 105.2, 108.1, 108.2, 120.6, 126.8, 127.7, 128.8, 134.1, 144.7, 146.2, 147.5, 149.8, 153.1, 166.7. HPLC : *t*_{R} *=* 7.5 min. MS : *m*/*z* 366 ([M+H]⁺).

*Preparation of 6-methyl-4-phenyl-2-thioxo-1,2,3,4-tetrahydropyrimidine-5-carboxylic acid **(20):*** Diethylamine (24.3 mmol) and tetrakis(triphenylphosphine)palladium (0.24 mmol) were added to a solution of **39** (2.4 mmol) in anhydrous tetrahydrofuran (5.5 mL) under argon. The reaction mixture was stirred at room temperature for 4h. Excess solvent was removed *in vacuo.* 0.5M KOH (20 mL) was added to the residue and the resulting suspension was filtered on Celite. The filtrate was acidified to pH 1-2 with HCl 37% and the resulting precipitate was collected by filtration, air-dried to afford **20** as a yellow powder (35%).

¹H NMR (400 MHz, *DMSO-d₆*) *δ* 2.28 (s, 3H), 5.15 (d, *J*= 3.6 Hz, 1H), 7.22-7.37 (m, 5H), 9.60 (s, 1H), 10.26 (s, 1H), 12.25 (bs, 1H). MS : *m*/*z* 249 ([M + H]⁺).

*Preparation of 4-methoxybenzyl 6-methyl-4-phenyl-2-thioxo-1,2,3,4-tetrahydropyrimidine-5-carboxylate **(52):*** A solution of **20** (0.83 mmol), 4-methoxybenzyl alcohol (1.67 mmol), EDCI (1.25 mmol) and DMAP (0.83 mmol) in anhydrous dimethylacetamide (6.0 mL) was heated under argon at 80 °C for 5 hours. *In vacuo* concentration of the reaction mixture followed by preparative HPLC purification (19x150 mm-5 µm, XBridge C18) afforded **52** as a white solid (18%). mp : 174-175 °C. TLC: *R*_{f} = 0.77 (cHex/EtOAc = 4/6).

¹H NMR (400 MHz, DMSO-*d₆*) *δ* 2.29 (s, 3H), 3.74 (s, 3H), 4.95 (d, *J =* 12.4 Hz, 1H), 5.00 (d, *J* = 12.0 *Hz,* 1H), 5.16 (d, *J* = 3.6 Hz, 1H), 6.86 (d, *J =* 8.4 Hz, 2H), 7.13 (d, *J* = 8.8 Hz, 2H), 7.15-7.17 (m, 2H), 7.28-7.32 (m, 3H), 9.66 (s, 1H), 10.37 (s, 1H). ¹³C NMR (100 MHz, DMSO-*d₆*) *δ* 17.6, 54.4, 55.5, 65.5, 100.8, 114.1, 126.8, 128.2, 128.5, 129.0, 130.1, 143.7, 146.0, 159.4, 165.4, 174.6. HPLC : *t*_{R} = 18.4 min. MS : *m*/*z* 369 ([M + H]⁺).

### III- Biological results

Some of the synthesized compounds were evaluated for their ability to inhibit iodide entrapment in FRTL5 cells. The IC₅₀ values were measured in at least two independent experiments. Sodium perchlorate was used as an assay control (IC₅₀ = 0.1 µM). The Compound 1 evaluated in the publication of N. Lecat-Guillet et al., ChemBioChem, 2008, 9, 889-895 (Compound ITB-9), is considered as the reference compound.

### III- 1) Protocols for biological evaluation

### Biological evaluation of the synthesized compounds:

The biological activity of each compound was determined in FRTL5 cells, using a non radioactive arsenic/cerium assay as described in F. Waltz et al., Anal. Biochem., 2012, 396, 91-95. Compound potency was expressed as IC₅₀, the concentration of compound necessary to achieve 50% inhibition of iodide uptake. Briefly, to FRTL5 cells at 70-90% confluence was added compound (200 µM, 10 µM, 0.5 µM, 25 nM, 1.2 nM, 60 pM, 30 pM, and 0.15 pM final), followed by NaI (10µM final). After 1 hour incubation at 20 ± 1 °C, supernatant was removed and the cells were immediately assayed for iodide content using the modified As/Ce Sandell-Kolthoff reaction. NaClO₄ was tested in each microplate as assay controls. The IC₅₀ values of all compounds were measured least thrice independently.

### Chemicals and solutions:

The uptake buffer consisted of Hank's balanced salt solution (HBSS) supplemented with HEPES (10 mM final). All chemicals were from Sigma-Aldrich unless otherwise stated. Ammonium cerium (IV) sulfate mother solution (42 mM): ammonium cerium (IV) sulfate hydrate (12.53 g, CAS 10378-47-9) was dissolved in water (200 mL). Concentrated H₂SO₄ (50 mL) was then added to the solution cooled with an ice bath. After cooling, the solution was diluted to 500 mL with water. This solution was stored in the dark at 4 °C for up to 6 months with no loss of activity. For bests results this solution was left to stand at 4 °C for 1 week before first use. This solution was diluted 4-fold with water prior to use.

Sodium arsenite (III) mother solution (96 mM): arsenic (III) oxide (4.75 g, CAS 1327-53-3) and NaCl (24 g) were dissolved in 2 M NaOH (50 mL). The mixture was then diluted to 500 mL with water and centrifuged to remove insoluble material. This solution was stored in the dark at room temperature for up to 6 months with no loss of activity. This solution was diluted 4-fold with water prior to use.

Iodide standards (S1 to S7): In a 100 mL volumetric flask, 29.98 mg of NaI was dissolved in water to make a 2 mM stock solution. The stock solution was diluted 100-fold in a 100 mL volumetric flask. This last solution was used for the preparation of NaI standards at 100, 200, 300, 400, 500, 600, 700 nM in water. These solutions were stored in the dark at room temperature for up to 2 months.

Stock solutions of tested compounds were prepared in DMSO (20 mM) and that of NaClO₄ in water (20 mM). These stock solutions were stored at 4 °C for up to 2 months. Sample dilutions: the day of the assay, a daughter plate (clear flat-bottomed 96-well polystyrene microplates, Costar 9017) was prepared from 20 mM stock solutions. NaClO4 (column 2) and DHPM samples (columns 4 to 11) were diluted at 10X the final concentration in uptake buffer.

### Cell culture:

FRTL5 cells were cultured as described in F. S. Ambesi-Impiombato et al., Proc. Natl. Acad. Sci. USA, 1980, 77, 3455-3459. Briefly, FRTL5 cells were cultured in Coon's modified F12 medium supplemented with 5% heat-inactivated fetal bovine serum (Invitrogen), 2 mM L-glutamine, 100 U/mL penicillin, 0.1 mg/mL streptomycin, 10 µg/mL insulin, 10 nM hydrocortisone, 10 ng/mL Gly-His-Lys acetate, 1 mU/mL TSH, 5 µg/mL transferrin at 37°C and 5% CO₂. For iodide uptake assays, 200 µL of FRTL5 cells with density of 250,000 cells/mL were dispensed in each well of clear flat-bottomed 96-well polystyrene microplates (Costar 3628) using the Multidrop 384 (ThermoFisher Scientific), and further cultured until confluence reached 80-90% (3-4 days).

### Iodide uptake:

The culture medium of FRTL5 monolayer cells at 80-90% confluence was replaced by uptake buffer (20 °C) via continuous aspiration/dispense cycle (600 µL) using a 96-needle head plate washer PW 384 (Tecan). The 96-needle head was set to a vertical position such that 80 µL of fresh uptake buffer remained in each well at the end of the cycle. The samples (10 µL each) from the daughter plate were transferred all at once to the assay plate using the 96-tip head pipettor Liquidator 96 (Mettler Toledo). Immediately after, 10 µL of a NaI solution at 100 µM was added to each well of the assay plate using the Liquidator 96. The assay plate was left to stand in the dark at 20 ± 1 °C for 60 min. The assay plate was then washed with cold (4°C) uptake buffer using the PW 384 plate washer and residual supernatant was immediately discarded by inverting the assay plate on absorbent paper.

### Iodide determination by As/Ce:

Sodium iodide standards (S1 to S7) and water (S0) were distributed in duplicate (100 µL each) in the first and last column of the assay plate. Water (100 µL) was added to the columns with the Multidrop 384. 100 µL of the ammonium cerium (IV) sulfate solution (10.5 mM) was distributed into the columns, followed by 100 µL of the sodium arsenite (III) solution (24 mM) using the Multidrop 384. The assay plate was left to stand at 20 ± 1 °C for 30 min. The absorbance at 420 nm (Abs₄₂₀) was immediately recorded on a SpectraMax Plus 384 (Molecular Devices). A calibration curve was prepared for each plate by plotting the logarithmic conversion of the means of Abs₄₂₀ (n = 2) vs iodide standard concentrations (S0 to S7). The iodide concentrations in the samples were determined after linear regression of the calibration curve. For IC₅₀ determination, experimental data were fitted by non-linear regression (least square) to the four-parameter sigmoidal Hill equation using an "in-house" application developed in Visual Basic for Excel (Microsoft).

### Cell viability:

Cell viability was tested according to a MTT-based assay (T. Mosmann, J. Immunol. Methods, 1983, 65, 55-63). Briefly, to FRTL5 cells at ~50% confluence was added compound (1 µM). Cell viability was determined at 24h end point before the addition of MTT (1.2 mg/mL). Absorbance at 570 nm was determined after 3h incubation at 37°C using a 96-well plate reader (Spectramax plus 384, Molecular Devices). Ouabain was tested as an assay control at eight distinct concentrations (2 µM-1 mM).

### III- 2) Results

**Table 1: Inhibitory activity (IC₅₀) against iodide uptake in FRTL5 cells. Variations at the R¹ position:**

| 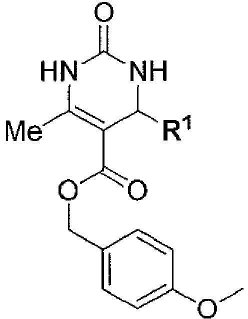 | | |
|---|---|---|
| Compound | R¹ | IC₅₀ (µM) |
| **21** | 3-Br-phenyl | 0.35 |
| **22** | 3-Cl-phenyl | 0.10 |
| **23** | 2-F-phenyl | 0.065 |
| **24** | 3-F-phenyl | 0.075 |
| **25** | 4-F-phenyl | 0.15 |
| **26** | *c*-propyl | 0.75 |
| **27** | 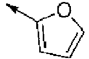 | 0.0032 |
| **28** | 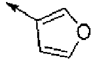 | 0.02 |
| **29** | 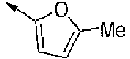 | 0.09 |
| **30** | 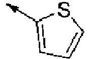 | 0.05 |
| **31** | 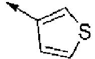 | 0.04 |

IC₅₀ values are averaged from three or four independent experiments. A standard deviation of 2-fold was judged acceptable. Arrow head indicate the point of attachment of R¹ to C-4 of the DHPM ring.

**Table 2: Inhibitory activity (IC₅₀) against iodide uptake in FRTL5 cells. Variations at the R⁵ position:**

| | | | |
|---|---|---|---|
| 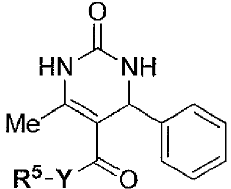 | | | |

| Compound | Y | R⁵ | IC₅₀ (µM) |
|---|---|---|---|
| **32** | O | benzyl | 0.19 |
| **33** | O | 2-Cl-benzyl | 0.35 |
| **34** | O | 4-Cl-benzyl | 0.3 |
| **35** | O | 4-F-benzyl | 0.2 |
| **36** | O | 2-Me-benzyl | 0.25 |
| **37** | O | 3-OMe-benzyl | 0.05 |
| **38** | O | 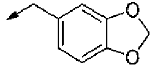 | 0.08 |

IC₅₀ values are averaged from three or four independent experiments. A standard deviation of 2-fold was judged acceptable. Arrow head indicate the point of attachment of R⁵ to Y (O or NH) heteroatom.

**Table 3: Inhibitory activity (IC₅₀) against iodide uptake in FRTL5 cells. Variations at the R² position:**

| | | |
|---|---|---|
| 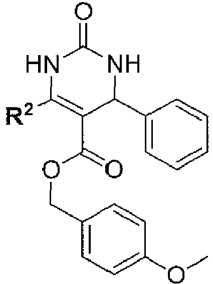 | | |

| Compound | R² | IC₅₀ (µM) |
|---|---|---|
| **40** | ethyl | 0.07 |
| **41** | *i*-butyl | 0.55 |
| **42** | *n*-pentyl | 0.35 |
| **43** | *c*-hexyl | 0.85 |
| **44** | phenyl | 0.95 |
| **45** | 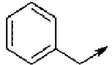 | 0.25 |
| **46** | 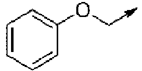 | 0.5 |
| **47** | 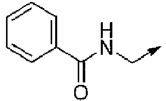 | 1.20 |
| **48** | 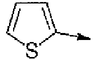 | 0.55 |
| **49** | 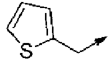 | 0.15 |

IC₅₀ values are averaged from three or tour independent experiments. A standard deviation of 2-fold was judged acceptable. Arrow head indicate the point of attachment of R² to C-6 of the DHPM ring.

**Table 4: Inhibitory activity (IC₅₀) against iodide uptake in FRTL5 cells. Simultaneous variations at X and the R³ and R⁴ position:**

| | | | | |
|---|---|---|---|---|
| 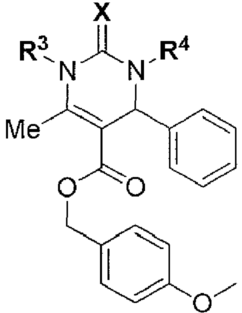 | | | | |

| Compound | R³ | X | R⁴ | IC₅₀ (µM) |
|---|---|---|---|---|
| **50** | methyl | O | H | 0.075 |
| **51** | methyl | O | methyl | 0.065 |
| **52** | H | S | H | 0.095 |

IC₅₀ values are averaged from three or four independent experiments. A standard deviation of 2-fold was judged acceptable. Arrow head indicate the point of attachment of R³ to N-1 and R⁴ to N-3 of the DHPM ring.

**Table 5: Inhibitory activity (IC₅₀) against iodide uptake in FRTL5 cells. Simultaneous variations at Y and the R¹, R³ R⁴ and R⁵ position:**

| | | | | | | |
|---|---|---|---|---|---|---|
| 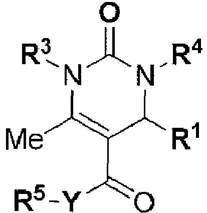 | | | | | | |

| Compound | R¹ | | Y | R⁴ | R⁵ | IC₅₀ (µM) |
|---|---|---|---|---|---|---|
| **53** | 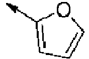 | methyl | O | H | 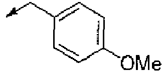 | 0.09 |
| **54** | 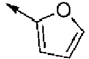 | methyl | O | methyl | 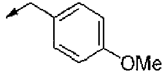 | 0.0016 |
| **55** | 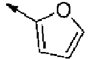 | H | O | H | 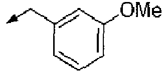 | 0.00057 |
| **56** | 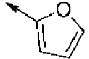 | methyl | O | H | 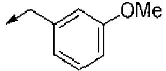 | 0.075 |
| **57** | 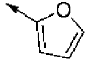 | methyl | O | methyl | 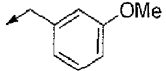 | 0.000065 |
| **58** | 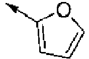 | methyl | O | methyl | 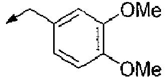 | 0.065 |
| **59** | 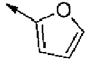 | H | O | H | 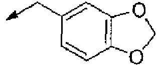 | 0.004 |
| **60** | 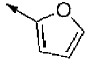 | methyl | O | H | 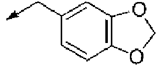 | 0.073 |
| **61** | 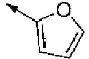 | methyl | O | methyl | 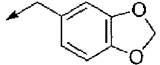 | 0.00085 |
| **62** | 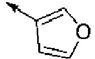 | H | O | H | 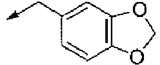 | 0.037 |
| **63** | 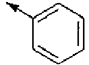 | H | NH | H | 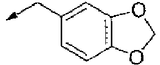 | 0.078 |

IC₅₀ values are averaged from two to four independent experiments. A standard deviation of 2-fold was judged acceptable. Arrow head indicate the point of attachment of R⁵ to Y (O or NH) heteroatom, R¹ to C-4, R³ to N-1 and R⁴ to N-3 of the DHPM ring.

Viability of the FRTL5 cells was also tested using a standard MTT assay in the presence of the above compounds at 1 µM. None of the DHPMs had an impact on cell growth at this concentration.

## Claims

1. A compound of general formula (I) below: 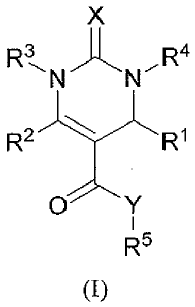 or a pharmaceutically acceptable salt thereof, wherein:
●X=OorS,
● Y=OorNH,
● R₁, R₂, R₃, R₄ and R₅, identical or different, are selected from hydrogen, optionally substituted C₁-C₂₀ linear or branched alkyl, alkoxy, alkenyl, alkynyl, cycloalkyl, aryl, arylalkyl, heteroalkyl, heteroaryl and heteroarylalkyl groups,
with the proviso that when X = O, Y = O, R₁ is a phenyl group, R₂ is a methyl group, R₃ and R₄ are hydrogen, R₅ is other than a 4-methoxybenzyl group,
for use as a medicament.

2. The compound of general formula (I) for its use according to Claim 1, wherein Y =O.

3. The compound of general formula (I) for its use according to Claim 1 or Claim 2, wherein R₁ is selected from C₁-C₆ linear or branched alkyl, cycloalkyl, arylalkyl, heteroalkyl, heteroaryl and heteroarylalkyl groups, optionally substituted with one or more groups independently selected from halogen, hydroxyl, cyano, nitro and C₁-C₇ alkoxy groups.

4. The compound of general formula (I) for its use according to Claim 3, wherein R₁ is selected from optionally substituted C₁-C₆ cycloalkyl, furane, thiophene, pyrrole, pyrazole, oxadiazole, oxazole, isoxazole, thiazole, isothiazole, and phenyl substituted with at least one halogen, and preferably R₁ is a furane.

5. The compound of general formula (I) for its use according to Claims 1 to 4, wherein R₂ is selected from C₁-C₆ linear or branched alkyl, phenyl, -CH₂-O-phenyl, benzyl, thiophene and -CH₂-thiophene,

6. The compound of general formula (I) for its use according to Claims 1 to 5, wherein R₃ is selected from hydrogen and C₁-C₆ linear or branched alkyl, and preferably R₃ is hydrogen or a methyl group.

7. The compound of general formula (I) for its use according to Claims 1 to 6, wherein R₄ is selected from hydrogen and C₁-C₆ linear or branched alkyl, and preferably R₄ is hydrogen or a methyl group.

8. The compound of general formula (I) for its use according to Claims 1 to 7, wherein R₅ is a benzyl group optionally substituted with one or more groups independently selected from halogen, methyl, hydroxyl, cyano, nitro and C₁-C₇ alkoxy groups.

9. The compound of general formula (I) for its use according to Claim 8, wherein R₅ is a methoxybenzyl group or a benzodioxolylmethyl group, such as piperonyl group.

10. The compound of general formula (I) for its use according to Claims 1 to 9, for the inhibition of sodium iodide symporter (NIS).

11. The compounds of general formula (I) for its use according to Claims 1 to 9, for the reduction of iodine transport and/or accumulation into NIS-expressing cells.

12. The compound of general formula (I) for its use according to Claims 1 to 9, for the *in vivo* diagnosis of NIS pathologies by functional imaging.

13. The compound of general formula (I) for its use according to Claims 1 to 9, for radioiodide decontamination after exposure to radioactive iodine species.

14. The compound of general formula (I) for its use according to Claims 1 to 9, for the prevention and/or the treatment of thyroid disorders, and more particularly of hyperthyroidism triggered by iodine overload, thyrotoxicosis, thyroiditis and toxic nodular goiter.

15. The compound of general formula (I) for its use according to Claims 1 to 9, for the prevention and/or the treatment of cancers, and more particularly of thyroid and breast cancers.

16. The compound of general formula (I) for its use according to Claims 1 to 9, for the prevention and/or the treatment of autoimmune diseases, and more particularly of Hashimoto and Basedow-Graves' diseases.

17. A pharmaceutical composition comprising at least one compound of formula (I) as defined according to Claims 1 to 9 as an active principle, and at least one pharmaceutically acceptable excipient.

18. A compound of general formula (I) below: 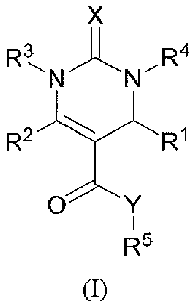 or a pharmaceutically acceptable salt thereof, wherein:
● X=OorS,
● Y = O or NH,
● R₁, R₂, R₃, R₄ and R₅, identical or different, are selected from hydrogen, optionally substituted C₁-C₂₀ linear or branched alkyl, alkoxy, alkenyl, alkynyl, cycloalkyl, aryl, arylalkyl, heteroalkyl, heteroaryl and heteroarylalkyl groups,
with the proviso that:
- when X = O, Y = O, R₁ is a phenyl group, R₂ is a methyl group, R₃ and R₄ are hydrogen, R₅ is other than hydrogen, a benzyl group, apiperonyl group, an ethyl group, a furan-2-ylmethyl group or a 4-methoxybenzyl group, or
- when X = S, Y = O, R₁ is a phenyl group, R₂ is a methyl group, R₃ and R₄ are hydrogen, R₅ is other than hydrogen or a propen-2-yl group.

19. The compound of general formula (I) according to Claim 18, wherein Y = O.

20. The compound of general formula (I) according to Claim 18 or Claim 19, wherein R₁ is selected from C₁-C₆ linear or branched alkyl, cycloalkyl, arylalkyl, heteroalkyl, heteroaryl and heteroarylalkyl groups, optionally substituted with one or more groups independently selected from halogen, hydroxyl, cyano, nitro and C₁-C₇ alkoxy groups.

21. The compound of general formula (I) according to Claims 18 to 20, wherein R₁ is selected from optionally substituted C₁-C₆ cycloalkyl, furane, thiophene, pyrrole, pyrazole, oxadiazole, oxazole, isoxazole, thiazole, isothiazole, and phenyl substituted with at least one halogen atom, and preferably R₁ is a furane.

22. The compound of general formula (1) according to Claims 18 to 21, wherein R₂ is selected from C₁-C₆ linear or branched alkyl, phenyl, -CH₂-O-phenyl, benzyl, thiophene and - CH₂-thiophene.

23. The compound of general formula (I) according to Claims 18 to 22, wherein R₃ is selected from hydrogen and C₁-C₇ linear or branched alkyl, and preferably R₃ is hydrogen or a methyl group.

24. The compound of general formula (I) according to Claims 18 to 23, wherein R₄ is selected from hydrogen and C₁-C₆ linear or branched alkyl, and preferably R₄ is hydrogen or a methyl group.

25. The compound of general formula (I) according to Claims 18 to 24, wherein R₅ is a benzyl group optionally substituted with one or more groups independently selected from halogen, methyl, hydroxyl, cyano, nitro and C₁-C₇ alkoxy groups.

26. The compound of general formula (I) according to Claim 25, wherein R₅ is a methoxybenzyl group or a benzodioxolylmethyl group, such as piperonyl group.
